(19) **Europäisches Patentamt**

**European Patent Office**

**Office européen des brevets**

(11) **EP 0 716 710 B1**

(12) **EUROPEAN PATENT SPECIFICATION**

(45) Date of publication and mention
of the grant of the patent:
**15.11.2000 Bulletin 2000/46**

(21) Application number: **94929135.5**

(22) Date of filing: **02.09.1994**

(51) Int. Cl.[7]: **C12N 15/86**, A61K 48/00,
C07K 14/075, C07K 14/045,
C12N 15/11, C12N 9/00,
C12N 5/10

(86) International application number:
**PCT/US94/09958**

(87) International publication number:
**WO 95/06744 (09.03.1995 Gazette 1995/11)**

(54) **METHODS OF SUPPRESSING IMMUNE RESPONSE BY GENE THERAPY**

VERFAHREN ZUR UNTERDRÜCKUNG DER IMMUNANTWORT DURCH GENTHERAPIE

PROCEDES DE SUPPRESSION DE LA REPONSE IMMUNITAIRE PAR THERAPIE GENIQUE

(84) Designated Contracting States:
**AT BE CH DE DK ES FR GB GR IE IT LI LU MC NL
PT SE**

(30) Priority: **03.09.1993 US 116828**

(43) Date of publication of application:
**19.06.1996 Bulletin 1996/25**

(73) Proprietor: **CHIRON CORPORATION
Emeryville, California 94608 (US)**

(72) Inventors:
 • **BARBER, Jack, R.
San Diego, CA 92129 (US)**
 • **WARNER, John, F.
San Diego, CA 92130 (US)**
 • **JOLLY, Douglas, J.
Leucadia, CA 92024 (US)**
 • **IRWIN, Michael, J.
San Diego, CA 92109 (US)**
 • **DUBENSKY, Thomas, W., Jr.
Del Mar, CA 92014 (US)**
 • **IBANEZ, Carlos, E.
San Diego, CA 92129 (US)**

(74) Representative:
**Goldin, Douglas Michael et al
J.A. KEMP & CO.
14 South Square
Gray's Inn
London WC1R 5LX (GB)**

(56) References cited:
**WO-A-93/05817          WO-A-93/09815
WO-A-93/14769          WO-A-94/16065**

**Description**

Technical Field

The present invention relates generally to the field of gene therapy and more specifically, to methods of preventing lymphocytes from recognizing and causing the destruction of tissue which express foreign genes.

Background of the Invention

The nature of human biology and disease depend on the function or dysfunction of specific genes. Consequently, certain gene products are required for normal growth, development, homeostasis, reproduction, immunity, and metabolism. Many genetic diseases caused by inheritance of defective genes result in the failure to produce normal gene products, for example, thalassemia, phenylketonuria, Lesch-Nyhan syndrome, severe combined immunodeficiency (SCID), hemophilia, A and B, cystic fibrosis, Duchenne's Muscular Dystrophy, inherited emphysema and familial hypercholesterolemia (Mulligan et al., Science 260:926, 1993; Anderson et al., Science 256:808, 1992; Friedman et al., Science 244:1275, 1989). Although genetic diseases may result in the absence of a gene product, endocrine disorders, such as diabetes and hypopituitarism, are caused by the inability of the gene to produce adequate levels of the appropriate hormone insulin and human growth hormone respectively.

Somatic gene therapy is a powerful method for treating these types of disorders. This therapy involves the introduction of normal recombinant genes into somatic cells so that new or missing proteins are produced inside the cells of a patient. For example, thalassemia is caused by an abnormality of the genes responsible for the production of hemoglobin. Introduction of a normal hemoglobin gene into red blood cell progenitors would result in the expression of normal levels of hemoglobin. This reconstitution of normal metabolic function overcomes the inability of the individual to synthesize an essential gene product and would be expected to reverse the disease process. In addition, this gene can also be used to alter the course of polygenic, multifactorial, and acquired diseases. In essence, somatic gene therapy acts as a drug delivery system for protein and RNA molecules.

One method of delivery of recombinant genes is DNA-mediated gene transfer or transfection. Transfection involves exposing cultured cells to various forms of DNA allowing them to take up these molecules and express the products they encode. Protocols for physical and chemical methods of uptake include calcium phosphate precipitates, direct microinjection of DNA into intact target cells (Williams et al., PNAS 88:2726, 1991), and electroporation, whereby cells suspended in a conducting solution are subjected to an intense electric field in order to transiently polarize the membrane allowing entry of macromolecules the size of genes. Other procedures include DNA ligand gene transfer where DNA is bound to a glycoprotein that binds to a receptor of a specific cell, for example, binding DNA to asialoglycoprotein which binds to asialoglycoprotein receptors on hepatocytes (Liang et al., J. Clin. Invest. 91:1241, 1993), DNA ligated to an inactivated adenovirus particle (Cotten et al., PNAS 89:6094, 1992), particle bombardment with DNA bound to particles, liposomes entrapping recombinant plasmids containing various genes of interest (Nabel et al., PNAS 89:5157, 1992; WO 93/00051), and spheroplast fusion whereby *E. coli* containing recombinant plasmids are stripped of their outer cell walls and fused to mammalian cells with polyethylene glycol (Cline et al., J. Pharmac. Ther. 29:69, 1985, and Friedmann et al., Science 244:1275, 1989). Recombinant gene expression in cultured cells by these methods may be short-lived with an efficiency of approximately 1% in suitable recipient cells, allowing stable integration into chromosomes.

Another method of delivering nucleic acids to animal cells is viral-mediated gene transfer or transduction. This greatly increases the transfer efficiency by using viral vectors capable of infecting virtually every cell in the target population. Viral transduction has been shown to be highly effective because viruses use selected and specific methods of cellular entry (Cline et al., Pharmac. Ther. 29:69, 1985). Many viruses may be utilized to administer vector constructs, including, for example, polio virus (Evans et al., Nature 339:385, 1989; Sabin et al., J. of Biol. Standardization 1:115, 1973) rhinovirus (Arnold et al., J. Cell. Biochem. L401, 1990); pox viruses, such as the canary pox virus or the vaccinia virus (Fisher-Hoch et al., PNAS 86:317, 1989, and Flexner et al., Ann. N.Y. Acad. Sci. 569:86, 1989; Flexner. et al., Vaccine 8:17, 1990); SV40 (Mulligan et al., Nature 277:108, 1979; Madzak et al, J. Gen. Vir. 73:1533, 1992); influenza virus (Luytjes et al., Cell 59:1107, 1989; McMicheal et al., The New England Journal of Medicine 309:13, 1983; and Yap et al., Nature 273:238, 1978); adenovirus (Berkner et al., Biotechniques 6:616, 1988, and Rosenfeld et al., Science 252:431, 1991); adeno-associated virus (Samulski et al., Journal of Virology 63:3822, 1989, and Mendelson et al., Virology 166:154, 1988); herpes virus (Kit et al., Adv. Exp. Med. Biol. 215:219, 1989); HIV (Buchschacher et al., J. Vir. 66:2731, 1992; EPO 386,882), measles virus (EPO 440, 219) Sindbis virus (Xiong et al., Science 234:1188, 1989) and coronavirus (Hemre et al., Proc. Soc. Exp. Biol. Med 121:190, 1966).

Many DNA viruses used for transduction present a number of problems, including the production of other viral proteins which may lead to pathogenesis or the suppression of the desired protein, the capacity of the vector to uncontrollably replicate in the host and the pathogenic control of such uncontrolled replication, the presence of wild-type virus which may lead to viremia, and the transitory nature of expression in these systems. These difficulties have thus far limited the use of viral vectors based on DNA viruses in the treatment of viral, cancerous, parasitic, genetic and other non-bacterial diseases.

By comparison, RNA containing viruses have shown promise for somatic gene therapy. Some of these vectors (e.g., retroviral vectors) convert the RNA gene of interest into DNA and integrate this sequence into the host genome. They are easily engineered to be replication defective, thereby rendering them incapable of forming new competent viral particles. In contrast to physical methods of recombinant gene transfer, viruses offer highly efficient entry into target cells, stable integration into the host genome (if desired), a conserved predictable structure for the introduced gene sequences, wide host range and low toxicity.

The major focus of somatic gene therapy is to introduce recombinant genes into different somatic sites, achieving stable and proper regulation of gene expression. Many such sites may be considered for targets of somatic gene therapy including fibroblasts, endothelial cells, epithelial cells, keratinocytes, thyroid follicular cells, and hematopoietic progenitor cells and various cell types that are in some way in a pathogenic state. Currently, bone marrow cells, hepatocytes , and T lymphocytes are the object of clinical trials (Ledley et al., Growth Gen. and Hor. 8:1, 1992). For example, recombinant retroviral vectors containing the hypoxanthine phosphoribosyl transferase (HPRT) gene responsible for Lesch-Nyhan syndrome have been transferred into bone marrow cells. The reconstitution of adequate levels of HPRT activity have proven that gene transfer can correct this specific metabolic defect in culture (Miller et al., Science 225:630, 1984 and Gruber et al., Science 230:1057, 1985). A number of other diseases have been proposed for treatment with gene therapy, including adenine deaminase deficiency, cystic fibrosis, $\alpha_1$-antitrypsin deficiency, Gaucher's syndrome, as well as non-genetic diseases. Bone marrow cells show promise because they are easily accessible and can be manipulated in vitro. Therefore, transformation of a discrete population of stem cells would support hematopoeisis and theoretically replenish the entire mass of marrow-derived elements. However, a disadvantage of this method is the resulting immune response to the introduced protein which is perceived as foreign.

Consequently, there is a need in the art for improved methods of suppressing the immune response following gene transfer, without the side effects or disadvantages of previously described methods. The present invention fulfills this need and further provides other related advantages.

Summary of the Invention

The present invention provides methods for transforming selected cells of an animal with a multivalent recombinant virus containing a sequence encoding a therapeutic protein of interest and a sequence responsible for inhibiting the antigen presentation pathway. Within one aspect of the present invention, a method is provided for suppressing the immune response within an animal, comprising transforming tissue cells of an animal with a multivalent recombinant vector construct that expresses a therapeutic protein and a second protein or active portion of the second protein capable of inhibiting MHC antigen presentation, such that an immune response against the therapeutic protein is suppressed. Within another aspect of the invention, a method is provided for suppressing an immune response within an animal by removing tissue cells from an animal; transforming the tissue cells with a multivalent recombinant vector construct that expresses a therapeutic protein and a second protein or active portion of the second protein capable of inhibiting MHC antigen presentation, and implanting the transformed tissue cells into an animal such that an immune response against the tissue cells is suppressed. Within one embodiment of the present invention, the multivalent recombinant vector construct directs the expression of a protein capable of binding $\beta_2$-microglobulin, such as HCMV-derived protein, H301. Within another embodiment, the recombinant vector construct directs the expression of a protein capable of binding the MHC class I heavy chain molecule intracellularly, such as E3/19K.

Within a related aspect of the present invention, the multivalent recombinant vector construct expresses a therapeutic protein and an antisense message capable of inhibiting MHC antigen presentation. Within various embodiments, the multivalent recombinant vector construct expresses a therapeutic protein and an antisense message which binds a conserved region of the MHC class I heavy chain transcripts, the $\beta_2$-microglobulin transcript, or the PSF-1 transporter protein transcript.

Within another aspect of the present invention, the multivalent recombinant vector construct expresses a therapeutic protein and transcribes a ribozyme capable of inhibiting MHC antigen presentation. Within various embodiments, the ribozyme is capable of cleaving a conserved region of MHC class I heavy chain transcripts, the $\beta_2$-microglobulin transcript, or the PSF-1 transporter protein transcript.

Within preferred embodiments of the present invention, the multivalent recombinant vector construct expresses at least one therapeutic protein selected from the group consisting of factor VIII, factor IX, hemoglobin, phenylalanine hydroxylase, adenosine deaminase, hypoxanthine-guanine phosphoribosyltransferase, $\alpha_1$-antitrypsin, dystrophin, cystic fibrosis, transmembrane conductance regulator, glucocerebrosidase, and the liver receptor to LDL.

Within the various aspects described above, the multivalent recombinant vector construct may be carried by a recombinant virus selected from the group consisting of togaviridae, picornaviridae, poxviridae, adenoviridae, parvoviridae, herpesviridae, paramyxoviridae and coronaviridae viruses. Within preferred embodiments, the multivalent recombinant vector construct is a recombinant viral vector construct. Particularly preferred constructs include recombinant retroviral vector constructs.

Tissue cells suitable for use within the present invention include fibroblast cells, bone marrow cells, endothelial cells, epithelial cells, muscle cells, neural cells, hepatocytes, follicular cells, hematopoietic progenitor cells, and lymphocytes. Within each of the general aspects discussed above, the multivalent recombinant vector construct may be administered *in vivo* or *ex vivo*.

Within still another related aspect of the present invention, pharmaceutical compositions are provided comprising tissue cells transformed with a multivalent recombinant vector construct and a physiologically acceptable carrier or diluent.

These and other aspects of the present invention will become evident upon reference to the following detailed description.

Detailed Description of the Invention

"Transforming" tissue cells refers to the transduction or transfection of tissue cells by any of a variety of means recognized by those skilled in the art, such that the transformed tissue cell expresses additional polynucleotides as compared to a tissue cell prior to the transforming event

"Implanting" refers to the insertion or grafting of tissue cells into a recipient animal such that at least a portion of the tissue cells are viable subsequent to implantation. The implanted tissue can be placed within tissue of similar function or of different function. For example, tissue cells from one animal may be removed and transformed with multivalent recombinant vector constructs before being "implanted" into another animal.

"Multivalent recombinant vector construct" or "vector construct" refers to an assembly which is capable of expressing sequences or genes of interest. In the context of protein expression, the vector construct must include promoter elements and may include a signal that directs polyadenylation. In addition, the vector construct preferably includes a sequence which, when transcribed, is operably linked to the sequences or genes of interest and acts as a translation initiation sequence. Preferably, the vector construct includes a selectable marker such as neomycin, thymidine kinase, hygromycin, phleomycin, histidinol, or dihydrofolate reductase (DHFR), as well as one or more restriction sites and a translation termination sequence. In addition, if the vector construct is used to make a retroviral particle , the vector construct must include a retroviral packaging signal and LTRs appropriate to the retrovirus used, provided these are not already present. The vector construct can also be used in combination with other viral vectors or inserted physically into cells or tissues as described below. As noted below, the multivalent recombinant vector construct includes a sequence that encodes at least one therapeutic protein of interest. The vector construct also includes a sequence that encodes a protein or active portion of a protein, antisense message, or ribozyme. Such sequences are designed to inhibit MHC antigen presentation, in order to suppress an immune response of class I restricted T-cells against transformed tissue cells.

In general, the multivalent recombinant vector constructs described herein are prepared by selecting a plasmid with a strong promoter, and appropriate restriction sites for insertion of DNA sequences of interest downstream from the promoter. As noted above, the vector construct may have a gene encoding antibiotic resistance for selection as well as termination and polyadenylation signals. Additional elements may include enhancers and introns with functional splice donor and acceptor sites.

The construction of multivalent recombinant vector constructs may require two promoters when two proteins are being expressed, because one promoter may not ensure adequate levels of gene expression of the second gene. In particular, where the vector construct expresses an antisense message or ribozyme, a second promoter may not be necessary. Within certain embodiments, an internal ribosome binding site (IRBS) or herpes simplex virus thymidine kinase (HSVTK) promoter is placed in conjunction with the second gene of interest in order to boost the levels of gene expression of the second gene (see Example 7). Briefly, with respect to IRBS, the upstream untranslated region of the immunoglobulin heavy chain binding protein has been shown to support the internal engagement of a bicistronic message (Jacejak et al., Nature 353:90, 1991). This sequence is small, approximately 300 base pairs, and may readily be incorporated into a vector in order to express multiple genes from a multicistronic message whose cistrons begin with this sequence.

Where the recombinant vector construct is carried by a virus, such constructs are prepared by inserting sequences of a virus containing the promoter, splicing, and polyadenylation signals into plasmids containing the desired gene of interest using methods well known in the art. The recombinant viral vector containing the gene of interest can replicate to high copy number after transduction into the target tissue cells.

Subsequent to preparation of the multivalent recombinant vector construct, it may be preferable to assess the ability of vector transformed cells to express the gene of interest and/or assess the down regulation of MHC presentation. In general, such assessments may be performed by Western blot, FACS analysis, or by other methods recognized by those skilled in the art.

Within preferred embodiments, the multivalent recombinant vector construct is carried by a retrovirus. Retroviruses are RNA viruses with a single positive strand genome which in general, are nonlytic. Upon infection, the retrovirus reverse transcribes its RNA into DNA, forming a provirus which is inserted into the host cell genome. Preparation of retroviral constructs for use in the present invention is described in greater detail in an application entitled "Recombinant Retroviruses" (U.S.S.N. 07/586,603, filed September 21, 1990) herein incorporated by reference. The retroviral genome can be divided conceptually into two parts. The "trans-acting" portion consists of the region coding for viral structural proteins, including the group specific antigen (gag) gene for synthesis of the core coat proteins; the pol gene for the synthesis of the reverse transcriptase and integrase enzymes; and the envelope (env) gene for the synthesis of envelope glycoproteins. The "cis-acting" portion consists of regions of the genome that is finally packaged into the viral particle. These regions include the packaging signal, long terminal repeats (LTR) with promoters and polyadenylation sites, and two start sites for DNA replication. The internal or "transacting" part of the cloned provirus is replaced by the gene of interest to create a "vector construct". When the vector construct is placed into a cell where viral packaging proteins are present (see U.S.S.N. 07/800,921), the transcribed RNA will be packaged as a viral particle which, in turn, will bud off from the cell. These particles are used to transduce tissue cells, allowing the vector construct to integrate into the cell genome. Although the vector construct express its gene product, the virus carrying it is replication defective because the trans-acting portion of the viral genome is absent. Various assays may be utilized in order to detect the presence of any replication competent infectious retrovirus. One preferred assay is the extended S$^+$L$^-$ assay described in Example 9. Preferred retroviral vectors include murine leukemia amphotropic or xenotropic, or VsVg pseudotype vectors (see WO 92/14829, incorporated herein by reference).

Recombinant vector constructs may also be developed and utilized with a variety of viral carriers including, for example, poliovirus (Evans et al., Nature 339:385, 1989, and Sabin et al., J. of Biol. Standardization 1:115, 1973) (ATCC VR-58); rhinovirus (Arnold et al., J. Cell. Biochem. L401, 1990) (ATCC VR-1110); pox viruses, such as canary pox virus or vaccinia virus (Fisher-Hoch et al., PNAS 86:317, 1989; Flexner et al., Ann. N.Y. Acad. Sci. 569:86, 1989; Flexner et al., Vaccine 8:17, 1990; U.S. 4,603,112 and U.S. 4,769,330; WO 89/01973) (ATCC VR-111; ATCC VR-2010); SV40 (Mulligan et al., Nature 277:108, 1979) (ATCC VR-305), (Madzak et al., J. Gen. Vir. 73:1533, 1992); influenza virus (Luytjes et al., Cell 59:1107, 1989; McMicheal et al., The New England Journal of Medicine 309:13, 1983; and Yap et al., Nature 273:238, 1978) (ATCC VR-797); adenovirus (Berkner et, al., Biotechniques 6:616, 1988, and Rosenfeld et al., Science 252:431, 1991) (ATCC VR-1); parvovirus such as adeno-associated virus (Samulski et al., J. Vir. 63:3822, 1989, and Mendelson et al., Virology 166:154, 1988) (ATCC VR-645); herpes simplex virus (Kit et al., Adv. Exp. Med. Biol. 215:219, 1989) (ATCC VR-977; ATCC VR-260); HIV (EPO 386,882, Buchschacher et al., J. Vir. 66:2731, 1992); measles virus (EPO 440,219) (ATCC VR-24); Sindbis virus (Xiong et al., Science 234:1188, 1989) (ATCC VR-68); and coronavirus (Hamre et al., Proc. Soc. Exp. Biol. Med. 121:190, 1966) (ATCC VR-740). It will be evident to those in the art that the viral carriers noted above may need to be modified to express a therapeutic gene of interest and proteins, antisense messages, or ribozymes capable of inhibiting MHC antigen presentation.

Once a multivalent vector construct has been prepared, it may be administered to a warm-blooded animal through a variety of routes, including both *ex vivo* and *in vivo* introduction. More specifically, within an *in vivo* context, naked DNA, or a multivalent recombinant vector construct containing a sequence that encodes a therapeutic protein and a sequence that encodes a second protein or active portion of the protein, an antisense message, or a ribozyme sequence capable of inhibiting MHC antigen presentation, can be injected into the interstitial space of tissues including muscle, brain, liver, skin, spleen, or blood (see WO 90/11092). Administration may also be accomplished by intravenous injection or direct catheter infusion into the cavities of the body (see WO 93/00051). Other representative examples of *in vivo* administration of vector constructs include transfection by various physical methods, such as lipofection (Felgner et al., PNAS 84:7413, 1989); microprojectile bombardment (Williams et al., PNAS 88:2726, 1991); liposomes (Wang et al., PNAS 84:7851, 1987); calcium phosphate (Dubensky et al., PNAS 81:7529, 1984); DNA ligand complexes (Wu et al., J. of Biol Chem. 264:16985, 1989; Cotten et al., PNAS 89:6094, 1992). As noted above, the vector construct may be carried by a virus such as vaccinia, Sindbis, or corona. Further, methods for administering a vector construct via a retroviral vector by direct injection are described in greater detail in an application entitled "Recombinant Retroviruses" (U.S.S.N. 07/586,603) herein incorporated by reference.

In addition, *ex vivo* procedures may be used in which cells are removed from an animal, transformed with a multivalent recombinant vector construct and placed into an animal. Cells that can be transformed include, but are not limited to, fibroblasts, endothelial cells, hepatocytes, epithelial cells, lymphocytes, keratinocytes, thyroid follicular cells and bone marrow cells. It will be evident that one can utilize any of the viral carriers noted above to introduce the multivalent recombinant vector construct into the tissue cells in an *ex vivo* context. Protocols for physical and chemical methods of uptake include calcium phosphate precipitation, direct microinjection of DNA into intact target cells, and electroporation whereby cells suspended in a conducting solution are subjected to an intense electric field in order to transiently polarize the membrane, allowing entry of macromolecules. Other suitable procedures include the use of DNA bound to ligand, DNA linked to an inactivated adenovirus (Cotten et al., PNAS 89:6094, 1992), bombardment with DNA bound to particles, liposomes entrapping recombinant vector constructs, and spheroplast fusion whereby *E. coli* containing recombinant viral vector constructs are stripped of their outer cell walls, and fused to animal cells using polyethylene glycol and viral transduction (Chine et al., Pharmac. Ther. 29:69, 1985, and Friedmann et al., Science 244:1275, 1989).

Within both *in vivo* and *ex vivo* procedures, tissue cells are transformed with a multivalent recombinant vector construct containing a sequence encoding at least one therapeutic protein and a sequence encoding a second protein or active portion of the protein, an antisense message or ribozyme, capable of inhibiting MHC antigen presentation. As discussed in more detail below, the multivalent recombinant vector construct may encode more than one protein (or active portion thereof), antisense message or ribozyme, in addition to one or more therapeutic proteins. In an *ex vivo* context, the transformed cells are implanted into a test animal, and monitored for gene expression as described in Example 14. Alternatively, the transformed cells may be tested *ex vivo*, using a human tissue culture system as described in Example 16. Protocols vary depending on the tissue cells chosen. Briefly, a multivalent recombinant vector construct carrying a sequence, the expression of which inhibits MHC class I presentation, is transformed into tissue cells. Preferable $10^5$ to $10^9$ tissue cells are transformed. The cells are cultured, and transformed cells may be selected by antibiotic resistance. Cells are assayed for gene expression by Western blot and FACS analysis, or other means. For example, as described in more detail below, bone marrow cells that have been transformed are implanted in an animal by intravenous administration of 2 to 3 x $10^7$ cells. *In vivo* expression of transformed cells may be detected by methods appropriate for the therapeutic protein utilized. In this regard, successful and sufficient suppression of the immune response will be indicated by persistent production of the protein.

As discussed above, the present invention provides methods and compositions suitable for inserting a sequence encoding a therapeutic protein and a sequence capable of inhibiting MHC antigen presentation in order to suppress the immune response of class I restricted cells within an animal. Briefly, CTL are specifically activated by the display of processed peptides in the context of self MHC molecules along with accessory molecules such as CD8, intercellular adhesion molecule -1 (ICAM-1), ICAM-2, (Singer, Science 255:1671, 1992; Rao, Crit. Rev. Immunol. 10:495, 1991), leukocyte functional antigen-1 (LFA-1) (Altmann et al., Nature 338:521, 1989), the B7/BB1 molecule (Freeman et al., J. Immunol. 143:2714, 1989), LFA-3 or other cell adhesion molecules. Antigenic peptide presentation in association with MHC class I molecules leads to CTL activation. Transfer and stable integration of specific sequences capable of expressing a therapeutic protein and products expected to inhibit MHC antigen presentation block activation of T-cells, such as $CD8^+$ CTL, and therefore prevent an immune response directed against cells expressing the therapeutic protein. A standard CTL assay is used to detect this response, as described in more detail in Example 13. Components of the antigen presentation pathway whose function may be inhibited in order to suppress effective MHC antigen presentation include the 45Kd MHC class I heavy chain, $\beta_2$-microglobulin, processing enzymes such as proteases, accessory molecules (as discussed above), chaperones, and transporter proteins such as PSF1.

Within one aspect of the present invention, a multivalent vector construct is provided which directs the expression of a therapeutic protein of interest and a protein or active portion of the protein capable of inhibiting MHC class I antigen presentation. Within the present invention, an "active portion" of a protein is that fragment of the protein which must be retained for biological activity. Such fragments or active domains can be readily identified by systematically removing nucleotide sequences from the protein sequence, transforming target cells with the resulting recombinant vector construct, and determining MHC class I presentation on the surface of cells using FACS analysis or other immunological assays, such as a CTL assay. These fragments are particularly useful when the size of the sequence encoding the entire protein exceeds the capacity of the viral carrier. Alternatively, the active domain of the MHC antigen presentation inhibitor protein can be enzymatically digested and the active portion purified by biochemical methods. For example, a monoclonal antibody that blocks the active portion of the protein can be used to isolate and purify the active portion of the cleaved protein (Harlow et al., Antibodies: A Laboratory Manual, Cold Springs Harbor, 1988).

Within one embodiment, the recombinant vector construct directs the expression of a protein or active portion of a protein that binds to newly synthesized MHC class I molecules intracellularly. This binding prevents migration of the MHC class I molecule from the endoplasmic reticulum, resulting in the inhibition of terminal glycosylation. This blocks transport of these molecules to the cell surface and prevents cell recognition and lysis by CTL. For instance, one of the products of the E3 gene may be used to inhibit transport of MHC class I molecules to the surface of the transformed cell. More specifically, E3 encodes a 19kD transmembrane glycoprotein, E3/19K, transcribed from the E3 region of the adenovirus 2 genome. Within the context of the present invention, a multivalent recombinant viral vector construct is administered directly or indirectly, and contains a gene encoding a therapeutic protein and the E3/19K sequence, which upon expression, produces the therapeutic protein and the E3/19K protein. The E3/19K protein inhibits the surface expression of MHC class I surface molecules, including those MHC molecules that have bound peptides of the therapeutic protein. Consequently, cells transformed by the vector evade an immune response against the therapeutic protein they produce. The construction of a representative multivalent recombinant vector construct in this regard is presented in Example 7.

Within another embodiment of the present invention, the multivalent recombinant vector construct directs the expression of a therapeutic protein and a protein or an active portion of a protein capable of binding $\beta_2$-microglobulin. Transport of MHC class I molecules to the cell surface for antigen presentation requires association with $\beta_2$-microglobulin. Thus, proteins that bind $\beta_2$-microglobulin and inhibit its association with MHC class 1 indirectly inhibit MHC class I antigen presentation. Suitable proteins include the H301 gene product. Briefly, the H301 gene, obtained from the human cytomegalovirus (CMV) encodes a glycoprotein with sequence homology to the $\beta_2$-microglobulin binding site on the heavy chain of the MHC class I molecule (Browne et al., Nature 347:770, 1990). H301 binds $\beta_2$-microglobulin, thereby preventing the maturation of MHC class I molecules, and renders transformed cells unrecognizable by cytotoxic T-cells, thus evading MHC class I restricted immune surveillance.

Other proteins, not discussed above, that function to inhibit or down-regulate MHC class I antigen presentation may also be identified and utilized within the context of the present invention. In order to identify such proteins, in particular those derived from mammalian pathogens (and, in turn, active portions thereof), a recombinant vector construct that expresses a protein or an active portion thereof suspected of being capable of inhibiting MHC class I antigen presentation is transformed into a tester cell line, such as BC. The tester cell lines with and without the sequence encoding the candidate protein are compared to stimulators and/or targets in the CTL assay. A decrease in cell lysis corresponding to the transformed tester cell indicates that the candidate protein is capable of inhibiting MHC presentation.

An alternative method to determine down-regulation of MHC class I surface expression is by FACS analysis. More specifically, cell lines are transformed with a recombinant vector construct encoding the candidate protein. After drug selection and expansion, the cells are analyzed by FACS for MHC class I expression and compared to that of non-transformed cells. A decrease in cell surface expression of MHC class I indicates that the candidate protein is capable of inhibiting MHC presentation (see, for instance, Example 12).

Within another aspect of the present invention, a multivalent vector construct is provided which directs the expression of at least one therapeutic protein and also transcribes an antisense message capable of inhibiting MHC class I antigen presentation. Briefly, oligonucleotides with nucleotide sequences complementary to the protein coding or "sense" sequence are termed "antisense". Antisense RNA sequences function as regulators of gene expression by hybridizing to complementary mRNA sequences and arresting translation (Mizuno et al., PNAS 81:1966, 1984; Heywood et al., Nucleic Acids Res. 14:6771, 1986). Antisense molecules comprising the entire sequence of the target transcript or any part thereof can be synthesized (Ferretti et al., PNAS 83:599, 1986), placed into vector constructs, and effectively introduced into cells to inhibit gene expression (Izant et al., Cell 36:1007, 1984). In addition, the synthesis of antisense RNA (asRNA) from DNA cloned in the inverted orientation offers stability over time while constitutive asRNA expression does not interfere with normal cell function.

Within one embodiment of the present invention, the multivalent recombinant vector construct transcribes a therapeutic gene of interest and an antisense message which binds to a conserved region of the MHC class I transcript(s), thereby inhibiting cell surface expression and MHC class I antigen presentation. One may identify such conserved regions through computer-assisted comparison of sequences representing different classes of MHC genes (for example, HLA A, B and C), available within DNA sequence databanks (e.g., Genbank). Conserved sequences are then identified through computer-assisted alignment for homology of the nucleotide sequences. The conserved region is a sequence having less than 50% mismatch, preferably less than 20% mismatch, per 100 base pairs between MHC class I genotypes.

Within another embodiment of the present invention, the multivalent recombinant vector construct expresses a therapeutic protein and an antisense message responsible for binding to the $\beta_2$-microglobulin transcript. This binding prevents translation of $\beta_2$-microglobulin protein and thereby inhibits proper assembly of the MHC class I molecule complex necessary for cell surface expression. Within a preferred embodiment, the nucleotide sequence for $\beta_2$-microglobulin is cloned into a vector construct in the reverse orientation. The proper antisense orientation is determined by restriction enzyme analysis.

Within still another embodiment of the present invention, the multivalent recombinant vector construct transcribes a therapeutic protein and an antisense message responsible for binding PSF1 transcript, a peptide transporter protein. Since this protein is necessary for the efficient assembly of MHC class I molecules, an antisense message to PSE1 transcript blocks the transport of processed antigenic peptide fragments to the endoplasmic reticulum (ER) prior to association with the $\beta_2$-microglobulin and MHC class I molecular complex. Within a preferred embodiment, the nucleotide sequence for PSF1 is prepared and inserted in the reverse orientation into the vector construct and determined by restriction enzyme analysis.

As discussed above, the sequences of other proteins involved in antigen presentation may also be identified, and used to design a multivalent recombinant vector construct capable of transcribing an antisense message that inhibits the antigen presentation pathway. More specifically, the nucleotide sequence of the gene encoding the protein is examined, and the identified sequence is used to synthesize an appropriate antisense message. It is preferable to use a sequence complimentary to a portion upstream or close to the start sequence of the target message. This allows the antisense sequence to bind to the mRNA preventing translation of a significant portion of the protein. Examples of such molecules are ICAM-1, ICAM-2, LFA-1, LFA-3 and B7/BB1. Down-regulation of MHC class I expression or antigen presentation may be assayed by FACS analysis or CTL assay, respectively, as described in Examples 13 and 15 or by other means as described above for proteins capable of inhibiting MHC class I presentation.

Within another aspect of the present invention a method is provided for suppressing an immune response within an animal by transforming selected cells of the animal with a multivalent recombinant vector construct which transcribes at least one therapeutic protein and a ribozyme responsible for the enzymatic cleavage of a component of the MHC antigen presentation pathway. Briefly, ribozymes are RNA molecules with enzymatic activity which are used to digest other RNA molecules. They consist of short RNA molecules possessing highly conserved sequence-specific cleavage domains flanked by regions which allow accurate positioning of the enzyme relative to the potential cleavage site in the desired target molecule. They provide highly flexible tools in inhibiting the expression and activation of specific genes (Haseloff et al., Nature 334:585, 1988). Custom ribozymes can be designed, provided that the transcribed sequences of the gene are known. Specifically, a ribozyme may be designed by first choosing the particular target RNA sequence and attaching complimentary sequences to the beginning and end of the ribozyme coding sequence. This ribozyme producing gene unit can then be inserted into a recombinant vector construct and used to transform tissue cells. Upon expression, the target gene is neutralized by complimentary binding and cleavage, guaranteeing permanent inactivation. In addition, because of their enzymatic activity, ribozymes are capable of destroying more than one target.

Within one embodiment of the present invention, multivalent recombinant vector constructs containing specific ribozymes are used to cleave the transcript of the conserved region of the MHC class I molecule in order to inhibit antigen presentation. Within another embodiment of the present invention, the multivalent recombinant vector construct encodes a therapeutic protein and a ribozyme responsible for the enzymatic cleavage of the $\beta_2$-microglobulin transcript. Specifically, a ribozyme with flanking regions complimentary to a sequence of the $\beta_2$-microglobulin message cleaves the transcript, thereby preventing protein translation and proper assembly of the MHC class I molecule complex. This inhibits transport of the MHC class I complex to the cell surface, thereby preventing antigen presentation.

Within still another embodiment of the present invention, the multivalent recombinant vector construct encodes a therapeutic protein and a ribozyme responsible for the enzymatic cleavage of the PSF1 transcript, thereby suppressing cell surface expression of the MHC class I molecules and preventing antigen presentation. More specifically, a ribozyme designed with flanking regions complimentary to a sequence of the PSF1 message cleaves the transcripts and inhibits transport of peptides to the ER, thereby preventing assembly of the MHC class I complex and antigen presentation.

As discussed above, it will be evident to those skilled in the art that the sequences of other proteins involved in antigen presentation may be identified and used to design a recombinant vector construct capable of transcribing a ribozyme that inhibits MHC antigen presentation. Down-regulation of MHC class I expression or antigen presentation may be assayed by FACS analysis or CTL assay, respectively, as described in Examples 13 and 15, or by other means as described above for proteins capable of inhibiting MHC class I presentation.

As noted above, the multivalent recombinant vector construct may express or transcribe more than one protein, antisense message or ribozyme capable of inhibiting MHC antigen presentation, in order to enhance the efficiency of the suppression of an immune response. Upon expression, the gene products increase the degree of interference with MHC antigen presentation by attacking a single component via two different routes or two different components via the same or different routes. The construction of multivalent recombinant vector constructs may require two promoters because one promoter may not ensure adequate levels of gene expression of the second gene. A second promoter, such as an internal ribozyme binding site (IRBS) promoter, or herpes simplex virus thymidine kinase (HSVTK) promoter placed in conjunction with the second gene of interest boosts the levels of gene expression of the second gene.

Within the preferred embodiments, in addition to at least one therapeutic protein, the multivalent vector construct expresses or transcribes at least two of the following components in any combination: (a) a protein or active portion of the proteins E3/19K or H301; (b) an antisense message that binds the transcript of a conserved region of the MHC class I heavy chain, $\beta_2$-microglobulin or PSF1 transporter protein; and (c) a ribozyme that cleaves the transcript of the proteins listed in (b) above. In addition, multivalent recombinant vector constructs are provided which express two proteins or active portions of proteins as described herein, two antisense messages, or two ribozymes. Within related embodiments, a number of specific combinations may be utilized in conjunction to sequences encoding at least one therapeutic protein to form a multivalent recombinant vector construct. For example, a multivalent recombinant vector construct may consist of a gene expressing E3/19K or H301 in combination with the antisense or ribozyme message for a conserved region of the MHC class I heavy chain, $\beta_2$-microglobulin, or PSF1 transporter protein.

Within the context of the present invention, sequences of interest include, but are not limited to, those responsible for the disorders; thalassemia, phenylketonuria, Lesch-Nyhan syndrome, severe combined immunodeficiency (SCID), hemophilia A and B, cystic fibrosis, Duchenne's muscular dystrophy, inherited emphysema, familial hypercholesterolemia, Gaucher's disease and various acquired diseases such as cancer and viral diseases. These genes code for hemoglobin constituents, phenylalanine hydroxylase, hypoxanthine-guanine phosphororibosyltransferase, adenosine deaminase, factors VIII and IX, cystic fibrosis transmembrane conductance regulator, dystrophin, $\alpha_1$-antitrypsin, the liver receptor for low density lipoprotein (LDL), and glucocerebrosidase, respectively. Sequences which encode transdominant mutated viral proteins that inhibit viral replication, mutant, non-human or synthetic ligands that bind an appropriate protein or molecule to inhibit disease progression, and prodrug activating enzymes (such as HSVTK) that allow elimination of cells by prodrug activation, may also be used within the present invention. In essence, any therapeutic protein which, when delivered via vector-based technology, is recognized as foreign by the patient's/animal's immune system may advantageously be used within the multivalent recombinant vector constructs described herein.

Within another aspect of the present invention, pharmaceutical compositions are provided comprising one of the above-described multivalent recombinant vector constructs, or a recombinant virus carrying the vector construct, such as retrovirus, poliovirus, rhinovirus, vaccinia virus, influenza virus, adenovirus, adeno-associated virus, herpes simplex virus, measles virus, coronavirus and Sindbis virus, in combination with a pharmaceutically acceptable carrier or diluent. The composition may be prepared either as a liquid solution, or as a solid form (e.g., lyophilized) which is resuspended in a solution prior to administration. In addition, the composition may be prepared with suitable carriers or diluents for either injection, oral, nasal or rectal administration or other means appropriate to the carrier. Carrying the vector construct is purified to a concentration ranging from 0.25% to 25%, and preferably about 5% to 20% before formulation. Subsequently, after preparation of the composition, the recombinant virus carrying the vector construct will constitute about 10 ng to 1 $\mu$g of material per dose, with about 10 times this amount of material present as copurified contaminants. Preferably, the composition is prepared in 0.1-1.0 ml of aqueous solution formulated as described below.

Pharmaceutically acceptable carriers or diluents are those which are nontoxic to recipients at the dosages and concentrations employed. Representative examples of carriers or diluents for injectable solutions include water, isotonic solutions which are preferably buffered at a physiological pH (such as phosphate-buffered saline or Tris-buffered saline) and containing one or more of mannitol, trehalose, lactose, dextrose, glycerol and ethanol, as well as polypeptides or proteins such as human serum albumin (HSA). One suitable composition comprises recombinant virus carrying a vector construct in 10 mg/ml mannitol, 1 mg/ml HSA, 20mM Tris pH=7.2 and 150mM NaCl. In this case, since the recombinant virus carrying the vector construct represents approximately 10 ng to 1 $\mu$g of material, it may be less than 1% of the total high molecular weight material, and less than 1/100,000 of the total material (including water). This composition is generally stable at -70°C for at least six months. It will be evident that substantially equivalent dosages of the multivalent recombinant vector construct may be prepared. In this regard, the vector construct will constitute 100 ng to 100 $\mu$g of material per dose, with about 10 times this amount present as copurified contaminants. Similarly, the transformed cells for implantation will constitute from $10^6$ to $10^{11}$ cells per dose.

The composition may be administered through a variety of routes (as discussed above), including intravenous (i.v.), subcutaneous (s.c.), or intramuscular (i.m.) injection. In this regard, it will be evident that the mode of administration will be influenced by the specific therapeutic application and the a vivo transformed cells (if any) utilized. For recombinant viruses carrying the vector construct, the individual doses normally used are $10^6$ to $10^{10}$ c.f.u. (e.g., colony forming units of neomycin resistance titered on HT1080 cells). These compositions are administered at one- to four-week intervals, for three or four doses (at least initially). Subsequent booster shots may be given as one or two doses after 6-12 months, and thereafter annually.

It will be evident to those skilled in the art that the dosage utilized will be influenced by a variety of factors, including the severity of the condition to be treated, the mechanism of action of the therapeutic protein, the body weight of the individual and the route of administration.

The following examples are offered by way of illustration and not by way of limitation.

## EXAMPLES

Example 1

PREPARATION OF MURINE RETROVIRAL PROVECTOR DNA

A. PREPARATION OF RETROVIRAL BACKBONE KT-3B

The Moloney murine leukemia virus (MoMLV) 5' long terminal repeat (LTR) EcoR I-EcoR I fragment, including gag sequences, from N2 vector (Armentano et al., J. Vir. 61:1647, 1987; Eglitas et al., Science 230:1395, 1985) in pUC31 plasmid is ligated into the plasmid SK+ (Stratagene, San Diego, CA). The resulting construct is called N2R5. The N2R5 construct is mutated by site-directed in vitro mutagenesis to change the ATG start codon to ATT preventing gag expression. This mutagenized fragment is 200 base pairs (bp) in length and flanked by Pst I restriction sites. The Pst I-Pst I mutated fragment is purified from the SK+ plasmid and inserted into the Pst I site of N2 MoMLV 5' LTR in plasmid pUC31 to replace the non-mutated 200 bp fragment. The plasmid pUC31 is derived from pUC19 (Stratagene, San Diego, CA) in which additional restriction sites Xho I, Bgl II, BssH II and Nco I are inserted between the EcoR I and Sac I sites of the polylinker. This construct is called pUC31/N2R5g$^M$.

The 1.0 kilobase (Kb) MoMLV 3' LTR EcoR I-EcoR I fragment from N2 was cloned into plasmid SK$^+$ resulting in a construct called N2R3$^-$. A 1.0 Kb Cla I-Hind III fragment is purified from this construct.

The Cla I-Cla I dominant selectable marker gene fragment from pAFVXM retroviral vector (Kriegler et al., Cell 38:483, 1984; St. Louis et al., PNAS 85:3150, 1988), comprising a SV40 early promoter driving expression of the neomycin phosphotransferase gene, is cloned into plasmid SK$^+$. A 1.3 Kb Cla I-BstB I gene fragment is purified from the SK$^+$ plasmid.

An alternative selectable marker, phleomycin resistance (Mulsant et al., Som. Cell and Mol. Gen. 14:243, 1988, available from Cayla, Cédex, FR) may be used to make the retroviral backbone KT-3C, for use in transforming genes to cells that are already neomycin resistant. The plasmid pUT507 (Mulsant et al., Som. Cell and Mol. Gen. 14:243, 1988) is digested with Nde I and the ends blunted with Klenow polymerase I. The sample is then further digested Hpa I, Cla I linkers ligated to the mix of fragments and the sample further digested with Cla I. The excess Cla I linkers are removed by digestion with Cla I and the 1.2 Kb Cla I fragment carrying the RSV LTR and the phleomycin resistance gene isolated by agarose gel electrophoresis followed by purification using Gene Clean (Bio101, San Diego, CA). This fragment is used in place of the 1.3 Kb Cla I-BstB I neomycin resistance fragment to give the backbone KT-3C.

The expression vector is constructed by a three part ligation in which the Xho I-Cla I fragment containing the gene of interest and the 1.0 Kb MoMLV 3' LTR Cla I-Hind III fragment are inserted into the Xho I-Hind III site of pUC31/N2R5g$^M$ plasmid. The 1.3 Kb Cla I-BstB I neo gene, or 1.2 Kb Cla I phleomycin, fragment is then inserted into the Cla I site of this plasmid in the sense orientation.

Example 2

A. CLONING OF E3/19K GENE INTO KT-3B

i. ISOLATION AND PURIFICATION OF ADENOVIRUS

The isolation and purification of adenovirus is described by Green et al., Methods in Enzymology 58: 425, 1979. Specifically, five liters of Hela cells (3-6 x 10$^5$ cells/ml) are infected with 100-500 plaque forming units (pfu) per ml of adenovirus type 2 (Ad2) virions (ATCC VR-846). After incubation at 37°C for 30-40 hours, the cells are placed on ice, harvested by centrifugation at 230g for 20 minutes at 4_ C, and resuspended in Tris-HCl buffer, pH 8.1. The pellets are mechanically disrupted by sonication and homogenized in trichlorotrifluoroethane prior to centrifugation at 1,000g for 10 min. The upper aqueous layer is removed and layered over 10 mls of CsCl (1.43 g/cm$^3$ ) and centrifuged in a SW27 rotor for 1 hour at 20,000 rpm. The opalescent adenovirus band is removed and adjusted to 1.34 g/cm$^3$ with CsCl and further centrifuged in a Ti 50 rotor for 16-20 hours at 30,000 rpm. The visible viral band in the middle of the gradient is removed and stored at 4°C until purification of adenoviral DNA.

ii. ISOLATION AND PURIFICATION OF ADENOVIRUS DNA

The adenovirus band is incubated with protease for 1 hour at 37°C to digest proteins. After centrifugation at 7,800g for 10 minutes at 4°C, the particles are solubilized in 5% sodium dodecyl sulfate (SDS) at room temperature for 30 minutes before being extracted with equal volumes of phenol. The upper aqueous phase is removed, re-extracted with phenol, extracted three times with ether, and dialyzed in Tris buffer for 24 hours. The viral Ad2 DNA is precipitated in ethanol, washed in ethanol, and resuspended in Tris-EDTA buffer, pH 8.1. Approximately 0.5 mg of viral Ad2 DNA is isolated from virus produced in 1.0 liter of cells.

iii. ISOLATION OF E3/19K GENE

The viral Ad2 DNA is digested with EcoR I (New England Biolabs, Beverly, MA) and separated by electrophoresis on a 1% agarose gel. The resulting 2.7 Kb Ad2 EcoR I D fragments, located in the Ad2 coordinate region 75.9 to 83.4, containing the E3/19K gene (Herisse et al., Nucleic Acids Research 8:2173, 1980, Cladaras et al., Virology 140:28, 1985) are eluted by electrophoresis, phenol extracted, ethanol precipitated, and dissolved in Tris-EDTA (pH 8.1).

iv. CLONING OF E3/19K GENE INTO KT-3B

The E3/19K gene is cloned into the EcoR I site of PUC1813. PUC1813 is prepared as essentially described by Kay et al., Nucleic Acids Research 15:2778, 1987 and Gray et al., PNAS 80:5842, 1983). The E3/19K is retrieved by EcoR I digestion and isolated fragment is cloned into the EcoR I site of phosphatase-treated pSP73 plasmid, (Promega, Madison, WI). This construct is designated SP-E3/19K. The orientation of the SP-E3/19K cDNA is verified by using appropriate restriction enzyme digestion and DNA sequencing. In the sense orientation, the 5' end of the cDNA is adjacent to the Xho I site of the pSP73 polylinker and the 3' end adjacent to the Cla I site. The Xho I-Cla I fragment containing the E3/19K cDNA in either sense or antisense orientation is retrieved from the SP-E3/19K construct and cloned into the Xho I-Cla I site of the KT-3B retroviral backbone. This construct is designated KT-3B/E3/19K.

B. CLONING OF PCR AMPLIFIED E3/19K GENE INTO KT-3B

i. PCR AMPLIFICATION OF E3/19K GENE

The Ad2 DNA E3/19K gene, including the amino terminal signal sequence, followed by the intraluminal domain and carboxy terminal cytoplasmic tail which allow the E3/19K protein to embed itself in the endoplasmic reticulum (ER), is located between viral nucleotides 28,812 and 29,288. Isolation of the Ad2 E3/19K gene from the viral genomic DNA is accomplished by PCR amplification, with the primer pair shown below:

The forward primer corresponds to the Ad2 nucleotide sequences 28,812 to 28,835.
(Sequence ID No. ____ )
    5'-3': TATATCTCCAGATGAGGTACATGATTTTAGGCTTG

The reverse primer corresponds to the Ad2 nucleotide sequences 29,241 to 29,213.
(Sequence ID No. ____ )
    5'-3': TATATATCGATTCAAGGCATTTTCTTTTCATCAATAAAAC

In addition to the Ad2 complementary sequences, both primers contain a five nucleotide "buffer sequence" at their 5' ends for efficient enzyme digestion of the PCT amplicon products. This sequence in the forward primer is followed by the Xho I recognition site and by the Cla I recognition site in the reverse primer. Thus, in the 5' to 3' direction, the E3/19K gene is flanked by Xho I and Cla I recognition sites. Amplification of the E3/19K gene from Ad2 DNA is accomplished with the following PCR cycle protocol:

| Temperature °C | Time (min) | No. Cycles |
|---|---|---|
| 94 | 2 | 1 |
| 94 | 0.5 | |
| 55 | 0.17 | 5 |
| 72 | 3.5 | |
| 94 | 0.5 | 30 |
| 70 | 3.5 | |
| 72 | 10 | 10 |

ii. LIGATION OF PCR AMPLIFIED E3/19K GENE INTO KT-3B

The E3/19K gene from the SK-E3/19K construct, approximately 780 bp in length, is removed and isolated by 1% agarose/TBE gel electrophoresis as described in Example 2Bi. The Xho I-Cla I E3/19K fragment is then ligated into the KT-3B retroviral backbone. This construct is designated KT-3B/E3/19K. It is amplified by transforming DH5α bacterial strain with the KT-3B/E3/19K construct. Specifically, the bacteria is transformed with 1-100 ng of ligation reaction mixture DNA. The transformed bacterial cells are plated on LB plates containing ampicillin. The plates are incubated overnight at 37°C, bacterial colonies are selected and DNA is prepared from them. The DNA is digested with Xho I and Cla I. The expected endonuclease restriction cleavage fragment sizes for plasmids containing the E3/19K gene are 780 and 1300 bp.

C. CLONING OF SYNTHESIZED E3/19K GENE INTO KT-3B

i. SYNTHESIS OF E3/19K GENE DNA

Chemical synthesis of synthetic DNA has been previously described (Caruthers et al., Methods in Enzymology 211:3, 1992). Sequences which encode the E3/19K gene are synthesized by the phosphotriester method on an Applied Biosystems Inc. DNA synthesizer, model 392 (Foster City, CA) using the PCR primers as the 5' and 3' limits and keeping the same Xho I and Cla I linkers on the ends. Short oligonucleotides of approximately 14-40 nucleotides in length are purified by polyacrylamide gel electrophoresis and ligated together to form the single-stranded DNA molecule (Ferretti et al., PNAS 83:599, 1986).

ii. SEQUENCING OF E3/19K GENE DNA

Fragments are cloned into the bacteriophage vectors M13mp18, and M13mp19, (GIBCO, Gaithersburg, MD ), for amplification of the DNA. The nucleotide sequence of each fragment is determined by the dideoxy method using the single-stranded M13mp18 and M13mp19 recombinant phage DNA as templates and selected synthetic oligonucleotides as primers. This confirms the identity and structural integrity of the gene.

iii. LIGATION OF SYNTHESIZED E3/19K GENE INTO KT-3B

The E3/19K gene is ligated into the KT-3B or KT-3C vector as previously described in Example 2Bii.

Example 3

CLONING OF AN ANTISENSE SEQUENCE OF A CONSERVED REGION OF MHC INTO KT-3C

A. CONSTRUCTION OF KT-3Cneoα MHC

The cDNA clone of the MHC class I allele CW3 (Zemmour et al., Tissue Antigens 39:249, 1992) is used as a template in a PCR reaction for the amplification of specific sequences, conserved across different human MHC (HLA) haplotypes, to be inserted into the untranslated region of the neomycin resistance gene of the KT-3B backbone vector.
The MHC class I allele CW3 cDNA is amplified between nucleotide sequence 147 to 1,075 using the following primer pairs:

The forward primer corresponds to MHC CW3 cDNA nucleotide sequence 147 to 166:
(Sequence ID No. _____ )
    5'-3': TATATGTCGACGGGCTACGTGGACGACACGC

The reverse primer corresponds to MHC CW3 cDNA nucleotide sequence 1,075 to 1,056:
(Sequence ID No. _____ )
    5'-3': TATATGTCGACCATCAGAGCCCTGGGCACTG

In addition to the MHC class I allele CW3 complementary sequences, both primers contain a five nucleotide "buffer sequence" at their 5' ends for efficient enzyme digestion of the PCR amplicon products. The buffer sequence is followed by the Hinc II recognition sequence in both primers. Generation of the MHC amplicon with the primers shown above is accomplished using the PCR protocol described in section 2Bi.. This protocol is modified by using Vent polymerase (New England Biolabs, Beverly, MA) and further modified to include 1 minute extension times instead of 3.5 minutes. The Vent polymerase generates amplicons with blunt ends. Alternatively, the forward and reverse primers may contain only the MHC CW3 complementary sequences.
The MHC CW3 cDNA 950 bp amplicon product is purified with Gene Clean (Bio101, San Diego, CA) and digested with Hinc II. The digested fragment, 938 bp is isolated by 1% agarose/TBE gel electrophoresis and purified with Gene Clean.
The MHC CW3 cDNA 938 bp fragment is inserted in the 3' untranslated region of the neomycin resistance gene in the antisense orientation. Specifically, the Hinc II recognition sequence at nucleotide sequence number 676 of the pBluescript II SK+ (pSK+) (Stratagene, San Diego, CA) plasmid is removed by digestion with Hinc II and Kpn I. The Kpn I 3' end is blunted with T4 DNA polymerase and the blunt ends are ligated. This plasmid is designated as pSKdlHII.

As described in Example 1A, the 1.3 Kb Cla I- Cla I dominant selectable marker gene fragment from pAFVXM or PUC507 retroviral vector is cloned into the Cla I site of pSKdlHII. This plasmid is designated as pSKdlHII/SVneo. The MHC CW3 cDNA 938 bp fragment is inserted in an antisense orientation into the Hinc II site of pSKdlHII/SVneo, located in the 3' untranslated region of the neomycin resistance gene. Confirmation that the MHC CW1 cDNA 938 bp fragment is present in the neomycin gene in an antisense orientation is determined by restriction endonuclease digestion and sequence analysis. This clone is designated as pSKdlHII/SVneo/αMHC.

Construction of KT3B/SVneo/αMHC is accomplished by a three way ligation, in which the Cla I 2.2 Kb SVneoαMHC fragment, and the 1.0 Kb MoMLV 3' LTR Cla I-Hind III fragment from N2R3⁻, are inserted between the Cla I and Hind III sites of pUC31/N2R5g^M plasmid as described in Example 1.

B. CONSTRUCTION OF KT3C/SVneo/YARNA/αMHC

High level MHC CW3 antisense RNA expression is accomplished by insertion of this sequence downstream of the Ad2 VARNA1 promoter. The Ad2 VARNA promoter-MHC antisense cDNA is assembled as a RNA polymerase III (pol III) expression cassette then inserted into the KT-3B or C backbone. In this pol III expression cassette, the Ad2 VARNA1 promoter is followed by the antisense αMHC cDNA, which in turn is followed by the pol III consensus termination signal.

The double stranded -30/+70 Ad2 VARNA1 promoter is chemically synthesized (Railey et al., Mol. Cell. Biol. 8:1147, 1988) and includes Xho I and Bgl II sites at the 5' and 3' ends, respectively.

The VARNA1 promoter, forward strand:
(Sequence ID No. ____ )
5'-3':   TCGAGTCTAGACCGTGCAAAAGGAGAGCCTGTAAGCGGGCACTCTTCC   GTGGTCTGGTGGATAAATTCGCAAGGGTATCATGGCGGACGACCGG GGTTCGAACCCCGGA

The VARNA1 promoter, reverse strand:
(Sequence ID No. ____ )
5'-3':   GATCTCCGGGGGTTCGAACCCCGGTCGTCCGCCATGATACCCTTGCGAA   TTTATCCACCAGACCACG-GAAGAGTGCCCGCTTACAGGCTCTCCTTT TGCACGGTCTAGAC

In order to form the double stranded VARNA1 promoter with Xho I and Bgl II cohesive ends, equal amounts of the single strands are mixed together in 10 mM MgCl₂, heated at 95°C for 5 min then cooled slowly to room temperature to allow the strands to anneal.

The MHC class I allele CW3 fragment, nucleotide sequence 653 to 854, from the plasmid pSKd1HII/SVneo/αMHC is amplified using the following primer pair:

The forward primer corresponds to nucleotide sequence 653 to 680:
5'-3': TATATCCTAGGTCTCTGACCATGAGGCCACCCTGAGGTG

The reverse primer corresponds to nucleotide sequence 854 to 827:
5'-3': TATATAGATCTACATGGCACGTGTATCTCTGCTCTTCTC

In addition to the MHC class I allele CW3 complementary sequences, both primers contain a five nucleotide "buffer sequence" at their 5' ends for efficient enzyme digestion of the PCR amplicon products. The buffer sequence is followed by the Avr II recognition sequence in the forward primer and by the Bgl II recognition sequence in the reverse primer, which allows insertion in an antisense orientation, relative to the Ad2 VARNA1 promoter in the pol III expression cassette. Generation of the MHC amplicon with the primers discussed above is accomplished with the PCR protocol described in Example 2Bi modified to include 0.5 minute extension times instead of 3.5 minutes.

The MHC CW3 cDNA 223 bp amplicon product is purified with Gene Clean (Bio101, San Diego, CA), then digested with AvrII and BglII, and isolated by 2% NuSeive-1% agarose/TBE gel electrophoresis. The 211 bp band is then excised from the gel and the DNA purified with Gene Clean.

The double stranded pot III consensus termination sequence is chemically synthesized (Geiduschek et al., Annu. Rev. Biochem. 57:873. 1988) and includes Avr II and Cla I sites at the 5' and 3' ends, respectively.

The pol III termination sequence, forward primer:
(Sequence ID No. ____ )
5'-3': CTAGGGCGCTTTTTGCGCAT

The pol III termination sequence, reverse primer:
(Sequence ID No. _____ )
    5'-3': CGATGCGCAAAAAGCGCC

In order to form the double stranded pol III transcription termination sequence with Avr II and Cla I cohesive ends, equal amounts of the single stands are mixed together in 10 mM MgCl$_2$, heated at 95°C for 5 min then cooled slowly to room temperature to allow the stands to anneal.

The pol III expression cassette for antisense αMHC class I allele CW3 is assembled in a four way ligation in which the Xho I-Bgl II Ad2 VARNA1 promoter fragment, the Bgl II-Avr II αMHC CW3 fragment, and the Avr II-Cla I transcription termination fragment, are cloned into pSKII$^+$ between the Xho I and Cla I sites. This construct is designated pSK/VARNA/αMHC.

Construction of KT3B/SVneo/VARNA/αMHC is accomplished in a two step ligation. The first step is a three way ligation in which the Xho I-Cla I VARNA/αMHC fragment and the 1.0 Kb MoMLV 3' LTR Cla I-Hind III fragment from N2R3$^-$, are inserted between the Xho I and Hind III sites of pUC31/N2R5g$^M$ plasmid as described in Example 1. This construct is designated KT3B/VARNA/αMHC. In the second ligation step the 1.3 Kb Cla I-BstB I SVneo fragment into the Cla I site of KT3B/VARNA/αMHC. This construct is designated KT3B/SVneo/VARNA/αMHC.

Example 4

CLONING A RIBOZYME THAT WILL CLEAVE A CONSERVED REGION OF MHC CLASS I HEAVY CHAIN INTO KT-3B

A. CONSTRUCTION OF pSK/VARNA/MHCHRBZ

In order to efficiently inhibit expression of MHC class I in transduced cells, a hairpin ribozyme with target specificity for the MHC class I allele is inserted into the KT3B/SVneo vector. The ribozyme is expressed at high levels from the Ad2 VARNA1 promoter. The MHC hairpin ribozyme (HRBZ) is inserted into the pol III pSK/VARNA/αMHC expression cassette described in Example 3.

The HRBZ and the MHC class I allele CW3 have the homologous sequence shown below:
(Sequence ID No. _____ )
    5'-3': GATG_AGTC_TCTCATCG

The HRBZ is designed to cleave after the A residue in the AGTC hairpin substrate motif contained in the target sequence. Following cleavage, the HRBZ is recycled and able to hybridize to, and cleave, other MHC class I RNA molecule.

Double stranded HRBZ as defined previously (Hampel et al., Nucleic Acids Research 18:299, 1990), containing a four base "tetraloop" 3 and an extended helix 4, with specificity for the MHC class I homologous sequence shown above, is chemically synthesized and includes Bgl II and Avr II sites at the 5' and 3' ends, respectively.

The MHC HRBZ, sense strand:
(Sequence ID No. _____ )
    5'-3': GATCTCGATGAGAAGAACATCACCAGAGAAACACACGGACTTC GGTCCGTGGTATATTACCTGGTAC

The MHC HRBZ, antisense strand:
(Sequence ID No. _____ )
    5'-3': CTAGGTACCAGGTAATATACCACGGACCGAAGTCCGTGTGTTTC TCTGGTGATGTTCTTCTCATCGA

In order to form the double stranded MHC class I specific HRBZ with Bgl II and Avr II cohesive ends, equal amounts of the single strands are mixed together in 10 mM MgCl$_2$, heated at 95°C for 5 min then cooled slowly to room temperature to allow the strands to anneal.

The pol III expression cassette for the MHC HRBZ is assembled by ligation of the chemically synthesized double stranded MHC class I specific HRBZ with Bgl II and Avr II cohesive ends into Bgl II and Avr II digested and CIAP treated pSK/VARNA/αMHC, in which the αMHC sequence has been removed from the expression vector. This plasmid is designated pSK/VARNA/MHCHRBZ and contains the Ad2 VARNA1 promoter followed by the MHC HRBZ, which in turn is followed by the pol III consensus termination sequence. The pol III expression components is flanked by Xho I and Cla I recognition sites.

B. CONSTRUCTION OF KT3B/SVneo/VARNA/MHCHRBZ

Construction of KT3B/SVneo/VARNA/MHCHRBZ is accomplished in a two step ligation. The first step is a three way ligation in which the Xho I-Cla I VARNA/MHCHRBZ fragment and the 1.0 Kb MoMLY 3' LTR Cla I-Hind III fragment from N2R3⁻, are inserted between the Xho I and Hind III sites of pUC31/N2R5g$^M$ plasmid described in Example 1. This construct is designated KT3B/VARNA/MHCHRBZ. In the second step, the 1.3 Kb Cla I-BstB I SVneo fragment is ligated into the Cla I site of KT3B/VARNA/MHCHRBZ. This construct is designated KT3B/SVneo/VARNA/MHCHRBZ.

Example 5

CLONING OF PSF1 ANTISENSE cDNA

A. CONSTRUCTION OF KT3C/SVneo/αPSF1

The cDNA clone of PSF1 (Spies et al., Nature 351:323, 1991; Spies et al., Nature 348:744, 1990) is used as a template in a PCR reaction for the amplification of specific sequences to be inserted into the KT-3B backbone vector, into the untranslated region of the neomycin resistant gene. The PSF1 cDNA is amplified between nucleotide sequence 91 to 1,124 using the following primer pairs:

The forward primer corresponds to nucleotide sequence 91 to 111:
(Sequence ID No. ____ )
5'-3': TATATGTCGACGAGCCATGCGGCTCCCTGAC

The reverse primer corresponds to nucleotide sequence 1,124 to 1,105:
(Sequence ID No. ____ )
5'-3': TATATGTCGACCGAACGGTCTGCAGCCCTCC

In addition to the PSF1 complementary sequences, both primers contain a five nucleotide "buffer sequence" at their 5' ends for efficient enzyme digestion of the PCR amplicon products. The buffer sequence is followed by the Hinc II recognition sequence in both primers. Generation of the PSF1 amplicon with the primers discussed above is accomplished with the PCR protocol described in Example 2Bi. This protocol is modified by using Vent polymerase (New England Biolabs, Beverly, MA) and further modified to include 1 minute extension times instead of 3.5 minutes. The Vent polymerase generates amplicons with blunt ends.

B. CONSTRUCTION OF KT3B/SVneo/VARNA/αPSF1

High level PSF1 antisense expression is accomplished by insertion of this sequence downstream of the Ad2 VARNA1 promoter. The Ad2 VARNA promoter-PSF1 antisense cDNA is first assembled as a pol III expression cassette then inserted into the KT-3B backbone. In this pol III expression cassette, the Ad2 VARNA1 promoter is followed by the antisense PSF1 cDNA, which in turn is followed by the pol III consensus termination signal.
The nucleotide sequence 91 to 309 of the PSF1 cDNA are amplified in a PCR reaction using the following primer pair:

The forward primer corresponds to nucleotide sequence 91 to 111:
(Sequence ID No. ____ )
5'-3': TATATCCTAGGGAGCCATGCGGCTCCCTGAC

The reverse primer corresponds to nucleotide sequence 309 to 288:
(Sequence ID No. ____ )
5'-3': TATATAGATCTCAGACAGAGCGGGAGCAGCAG

In addition to the PSF1 complementary sequences, both primers contain a five nucleotide "buffer sequence" at their 5' ends for efficient enzyme digestion of the PCR amplicon products. The buffer sequence is followed by the Avr II recognition sequence in the forward primer and by the Bgl II recognition sequence in the reverse primer, which allows insertion in an antisense orientation, relative to the Ad2 VARNA1 promoter in the RNA polymerase III expression cassette. Generation of the PSF1 amplicon with the primers described above is accomplished with the PCR protocol described in Example 2Bi modified to include 0.5 minutes extension times instead of 3.5 minutes.

The MHC CW3 cDNA 240 bp amplicon product is purified with Gene Clean (Bio101, San Diego, CA), then digested with Avr II and Bgl II, and isolated by 2% NuSeive-1% agarose/TBE gel electrophoresis. The 211 bp band is then excised from the gel and purified with Gene Clean.

Construction of KT3B/SVneo/VARNA/αPSF1 is accomplished in two step ligation. The first step is a three-way ligation in which the Xho I-Cla I VARNA/αPSF1 fragment and the 1.0 Kb MoMLV 3' LTR Cla I-Hind III fragment from N2R3⁻, are inserted between the Xho I and Hind III sites of pUC31/N2R5g^M plasmid as described in Example 1. This construct is designated as KT3B/VARNA/αPSF1. In the second ligation step, the 1.3 kb Cla I-BstB I SVneo fragment is ligated into the Cla I site of KT3B/VARNA/αPSF1. This construct is designated KT3B/SVneo/VARNA/αPSF1.

Example 6

CLONING A RIBOZYME THAT WILL CLEAVE A CONSERVED REGION OF PSF1 INTO KT-3B

A. CONSTRUCTION OF pSK/VARNA/PSF1HRBZ

In order to efficiently inhibit expression of PSF1 in transduced cells, a hairpin ribozyme with target specificity for the PSF1 RNA is inserted into the KT3B/SVneo vector. The ribozyme is expressed at high levels from the Ad2 VARNA1 promoter. The PSF1 hairpin ribozyme (HRBZ) is inserted into the pol III pSK/VARNA/αMHC expression cassette described in Example 3. The PSF1 HRBZ-pol III expression cassette is then inserted into the KT3B/SVneo backbone vector.

The HRBZ and the PSF1 RNA have the homologous sequence shown below:
(Sequence ID No. _____ )

    5'-3': GCTC<u>TGTC</u>TGGCCAC

The HRBZ is designed to cleave after the T residue in the <u>T</u>GTC hairpin substrate motif contained in the target sequence. Following cleavage, the HRBZ is recycled and able to hybridize to, and cleave, other PSF1 RNA molecule.

Double stranded HRBZ as defined previously (Hampel et al., Nucleic Acids Research 18:299, 1990), containing a four base "tetraloop" 3 and an extended helix 4, with specificity for the PSF1 homologous sequence shown above, is chemically synthesized and includes Bgl II and Avr II sites at the 5' and 3' ends, respectively.

The PSF1 HRBZ, sense strand:
(Sequence ID No. _____ )

    5'-3': GATCTGTGGCCAGACAGAGCACCAGAGAAACACACGGACTTCG  GTCCGTGGTATATTACCTGGTAC

The PSF1 HRBZ, antisense strand:
(Sequence ID No. _____ )

    5'-3': CTAGGTACCAGGTAATATACCACGGACCGAAGTCCGTGTGTTTC    TCTGGTGCTCTGTCTGGCCACA

In order to form the double stranded PSF1 specific HRBZ with Bgl II and Avr II cohesive ends, equal amounts of the single strands are mixed together in 10 mM MgCl₂ heated at 95°C for 5 min then cooled slowly to room temperature to allow the strands to anneal.

The pol III expression cassette for the PSF1 HRBZ is assembled by ligation of the chemically synthesized double stranded PSF1 specific HRBZ with Bgl II and Avr II cohesive ends into Bgl II and Avr II digested and CIAP treated pSK/VARNA/αMHC, in which the αMHC sequence has been gel purified away from the pol III expression vector. This plasmid is designated pSK/VARNA/PSF1HRBZ and contains the Ad2 VARNA1 promoter followed by the PSF1 HRBZ, which in turn is followed by the pol III consensus termination sequence. The pol III expression component is flanked by Xho I and Cla I recognition sites.

B. CONSTRUCTION OF KT3B/SVneo/VARNA/PSF1HRBZ

Construction of KT3B/SVneo/VARNA/MHCHRBZ is accomplished in a two step ligation. The first step is a three way ligation in which the Xho I-Cla I VARNA/PSF1HRBZ fragment and the 1.0 Kb MoMLV 3' LTR Cla I-Hind III fragment from N2R3⁻, are inserted between the Xho I and Hind III sites of pUC31/N2R5g^M plasmid as described in Example 1. This construct is designated KT3B/VARNA/PSF1HRBZ. In the second ligation step, the 1.3 Kb Cla I-BstB I SVneo fragment is ligated into the Cla I site of KT3B/VARNA/PSF1HRBZ. This construct is designated KT3B/SVneo/VARNA/PSF1HRBZ.

Example 7

CONSTRUCTION OF THE MULTIVALENT RECOMBINANT RETROVIRAL VECTOR KT3B-GC/E3/19K

Gaucher disease is a genetic disorder that is characterized by the deficiency of the enzyme glucocerebrosidase. This enzyme deficiency leads to the accumulation of glucocerebroside in the lysosomes of all cells in the body. However, the disease phenotype is manifested only in macrophages, except in the very rare neuronpathic forms of the disease. The disease usually leads to enlargement of the liver and spleen and may also cause lesions in the bones. (Beutler et al., Science 256:794, 1992; and Scriver et al., The Metabolic Basis of Inherited Disease, 6th ed., 2:1677). This type of therapy is an example of a single gene replacement therapy which would provide a deficient cellular enzyme.

i. Construction of KT3B-GC

A glucocerebrosidase (GC) cDNA clone containing an Xho I restriction enzyme site 5' of the cDNA coding sequence and a Cla I restriction enzyme site 3' of the cDNA coding sequence is first generated. The clone is generated by digesting pMFG-GC (Ohashi et al., PNAS 89:11332, 1992, Nolta et al., Blood, 75:787, 1991) with Nco I (New England Biolabs, Beverly, MA), blunted with Vent DNA polymerase (New England Biolabs, Beverly, MA), then ligated with Xho I linkers. The plasmid is then digested with Bam HI (New England Biolabs, Beverly, MA), blunted with Vent DNA polymerase, then ligated to Cla I linkers. The fragment is then digested with Xho I and Cla I and ligated in a three part ligation in which the Xho I -Cla I GC fragment and the 1.0 Kb MoMLV 3' LTR Cla I-Hind III fragment are inserted into the Xho I-Hind III site of pUC31/N2R5g$^M$ plasmid, Example 1. This construct is designated as KT3B-GC.

ii. Construction of KT3-GC/E3/19K

The plasmid pBS-ECAT (Kräusslich et al., J. Vir. 61:2711, 1987, Gorman et al., Mol. Cell. Bio. 2:1044, 1982, Jang et al., J. Virol 63:1651, 1989) includes the 5' nontranslated region of encephalomyocarditis virus, EMCV (ATTC No. VR-129B), from nucleotides 260-848 of the viral genome, which contains the IRES. EMCV nucleotides 260-827 are amplified from pBS-ECAT by PCR, using the following primer pair:

The forward primer contains the IRBS and a Bst BI endonuclease site.
(Sequence ID No. ____ )
       5'-3': TATATTTCGAACCCCCCCCCCCCCCCCAACG

The reverse primer contains the IRBS and a Sal I endonuclease site.
(Sequence ID No. ____ )
       5'-3': TATATGTCGACCTTACAATCGTGGTTTTCAAAGG

The amplicon resulting from amplification with the forward primer and reverse primer is flanked by Bst BI and Sal I recognition sites, inside a 5 bp "buffer sequence". After PCR amplification, the amplicon is digested with Sal I and ligated with the plasmid KT3B-E3/19K, Example 2, previously digested with Xho I. After ligation, the linearized plasmid is then digested with Bst B I and Cla I releasing a Bst BI and Cla I fragment containing the IRBS sequences linked to the E3/19K sequences. This Bst BI-Cla I fragment is then ligated into the KT3B-GC construct, previously digested with Cla I. This plasmid is known as KT3B-GC/E3/19K.

iii. Construction of KT3B-GC/E3/19KαMHC

A variation of the retroviral vector KT3B-GC/E3/19K can also be constructed containing both GC and E3/19K sequences but in addition contains anti-sense sequences specific for a conserved region between the three class I MHC alleles A2, CW3 and B27, Examples 2 and 3. This vector, known as KT3B-GC/E3/19K/αMHC, is designed to incorporate the MHC class I anti-sense sequences at the 3' end of the E3/19K sequence which would be expressed as a chimeric molecule. The retroviral vector, KT3B-GC/E3/19K/αMHC, can be constructed by ligating a Cla I digested PCR amplified product containing the MHC anti-sense sequences into the Cla I site of the KT3B-GC/E3/19K vector. More specifically, the cDNA clone of the MHC class I allele CW3 (Zemmour et al., Tissue Antigens 39:249, 1992) is amplified by PCR between nucleotides 653 and 854 using the following primer pair:

The forward primer of αMHC is:
(Sequence ID No. ____ )

5'-3': ATTATCGATTCTCTGACCATGAGGCCACCCTGAGGTG

The reverse primer of αMHC is:

(Sequence ID No. _____ )

5'-3': ATTAATCGATACATGGCACGTGTATCTCTGCTCTTCTC

The primer pairs are flanked by Cla I restriction enzyme sites in order to insert an amplified Cla I digested product into the partially pre-digested KT3B-GC/E3/19K vector in the anti-sense orientation. By placing the Cla I fragment in the reverse orientation the vector will express the negative anti-sense strand upon transcription.

iv. Construction of the KT3B-GC/αMHC

One further example of a GC retroviral vector with MHC class I down regulating capabilities is the KT3B-GC/αMHC construct which contains the same anti-sense sequences specific for the consented regions between the three MHC class I alleles described above. In this example the MHC anti-sense sequence is designed to be incorporated at the 3' end of the therapeutic gene in the context of the full KT-3B backbone which includes the neomycin selectable marker. More specifically the Cla I-BstB I neomycin gene fragment as described in Example 1 is inserted, in the sense orientation, into the KT3B-GC construct as described above, pre-digested and treated with Cla I and calf intestinal alkaline phosphatase. Once a clone is selected by restriction enzyme analysis, the same Cla I digested PCR amplified product containing conserved MHC class I sequences described in the construction of KT3B-GC/E3/19K/αMHC, is ligated into KT3B-GC/Neo vector, pre-digested and treated with Cla I and calf intestinal alkaline phosphatase (CIAP), to create the KT3B-GC/αMHC expression vector.

Example 8

TRANSDUCTION OF PACKAGING CELL LINES DA WITH THE MULTIVALENT RECOMBINANT RETROVIRAL VECTOR KT3B-GC/E3/19K

A. PLASMID DNA TRANSFECTION

293 2-3 cells (a cell line derived from 293 cells ATCC No. CRL 1573, WO 92/05266) 5 x $10^5$ cells are seeded at approximately 50% confluence on a 6 cm tissue culture dish. The following day, the media is replaced with 4 ml fresh media 4 hours prior to transfection. A standard calcium phosphate-DNA coprecipitation is performed by mixing 10.0 μg of KT3B-GG/E3/19K plasmid and 10.0 μg MLP G plasmid with a 2M $CaCl_2$ solution, adding a 1x Hepes buffered saline solution, pH 6.9, and incubating for 15 minutes at room temperature. The calcium phosphate-DNA coprecipitate is transferred to the 293 2-3 cells, which are then incubated overnight at 37°C, 5% $CO_2$. The following morning, the cells are rinsed three times in 1x PBS, pH 7.0. Fresh media is added to the cells, followed by overnight incubation at 37°C, 10% $CO_2$. The following day, the media is collected off the cells and passed through a 0.45 μ filter. This supernatant is used to transduce packaging and tumor cell lines. Transient vector supernatant for other vectors are generated in a similar fashion.

B. PACKAGING CELL LINE TRANSDUCTION

DA cells (an amphotropic cell line derived from D-17 cells ATCC No. 183, WO 92/05266) are seeded at 5 x $10^5$ cells/10 cm dish. Approximately 0.5 ml of the freshly collected 293 2-3 supernatant (or supernatant that has been stored at -70° C) is added to the DA cells. The following day, Phleomycin is added to these cells and a drug resistant pool is generated over a period of a week. This pool of cells is dilution cloned to yield a single cell per well of 96 well plates. Twenty-four clones are expanded to 24 well plates, then to 6 well plates, at which time cell supernatants are collected for titering. DA clones are selected for vector production and called DA-GC/E3/19K. Vector supenatants are collected from 10cm confluent plates of DA-GC/E3/19K clones cultured in normal media containing polybrene or protamine sulfate. Alternatively, vector supernatant can be harvested from bioreactors or roller bottles, processed and purified further before use.

For those vectors without a drug resistance marker, or with a marker already in the packaging cell line selection of stably transduced clones must be performed by dilution cloning the DA transduced cells one to two days after transducing the cells with 293 2-3 generated supernatant. The dilution clones are then screened for the presence of both glucocerebrosidase and E3/19K expression by using reverse transcription of messenger RNA, followed by amplification of the cDNA message by the polymerase chain reaction, a procedure is known as the RT-PCR. A commercial kit for RT-PCR is available through Invitrogen Corp. (San Diego, CA). RT-PCR should be performed on clones which have been propagated for at least 10 days and approximately 50 to 100 clones will need to be screened in order to find a reasonable number of stably transformed clones. In order to perform RT-PCR, specific primers will be required for each message to be amplified. Primers designed to amplify a 521 bp product for glucocerebrosidase and a 401 bp product for E3/19K message screening are as follows:

Screening primers for glucocerebrosidase:
(Sequence ID No. ____ )
5'-3': TTTCTGGCTCCAGCCAAAGCCACCCTAGGGGAG
(Sequence ID No. ____ )
5'-3': AATGGAGTAGCCAGGTGAGATTGTCTCCAGGAA

Screening primers for E3/19K are:
(Sequence ID No. ____ )
5'-3': ATGAGGTACATGATTTTAGGCTTG
(Sequence ID No. ____ )
5'-3': TCAAGGCATTTTCTTTTCATCAATAAAAC

Example 9

DETECTION OF REPLICATION COMPETENT RETROVIRUSES

The extended $S^+L^-$ assay determines whether replication competent, infectious virus is present in the supernatant of the cell line of interest. The assay is based on the empirical observation that infectious retroviruses generate foci on the indicator cell line $MiCl_1$ (ATCC CCL 64.1). The $MiCl_1$ cell line is derived from the Mv1Lu mink cell line (ATCC CCL 64) by transduction with Murine Sarcoma Virus (MSV). It is a non-producer, non-transformed, revertant clone containing a murine sarcoma provirus that forms sarcoma ($S^+$) indicating the presence of the MSV genome but does not cause leukemia ($L^-$) indicating the absence of replication competent virus. Infection of $MiCl_1$ cells with replication competent retrovirus "activates" the MSV genome to trigger "transformation" which results in foci formation.

Supernatant is removed from the cell line to be tested for presence of replication competent retrovirus and passed through a 0.45 μ filter to remove any cells. On day 1, Mv1Lu cells are seeded at 1 x $10^5$ cells per well (one well per sample to be tested) of a 6 well plate in 2 ml DMEM, 10% FBS and 8 μg/ml polybrene. Mv1Lu cells are plated in the same manner for positive and negative controls on separate 6 well plates. The cells are incubated overnight at 37°C, 10% $CO_2$. On day 2, 1.0 ml of test supernatant is added to the Mv1Lu cells. The negative control plates are incubated with 1.0 ml of media. The positive control consists of three dilutions (200 focus forming units (ffu), 20 ffu and 2 ffu each in 1.0 ml media) of MA virus (Miller et al., Molec. and Cell Biol. 5:431, 1985) which is added to the cells in the positive control wells. The cells are incubated overnight. On day 3, the media is aspirated and 3.0 ml of fresh DMEM and 10% FBS is added to the cells. The cells are allowed to grow to confluency and are split 1:10 on day 6 and day 10, amplifying any replication competent retrovirus. On day 13, the media on the Mv1Lu cells is aspirated and 2.0 ml DMEM and 10% FBS is added to the cells. In addition, the $MiCl_1$ cells are seeded at 1 x $10^5$ cells per well in 2.0 ml DMEM, 10% FBS and 8 μg/ml polybrene. On day 14, the supernatant from the Mv1Lu cells is transferred to the corresponding well of the $MiCl_1$ cells and incubated overnight at 37°C, 10% $CO_2$. On day 15, the media is aspirated and 3.0 ml of fresh DMEM and 10% FBS is added to the cells. On day 21, the cells are examined for focus formation (appearing as clustered, refractile cells that overgrow the monolayer and remain attached) on the monolayer of cells. The test article is determined to be contaminated with replication competent retrovirus if foci appear on the $MiCl_1$ cells.

Example 10

TRANSDUCTION OF CELL LINES WITH E3/19K RETROVIRAL VECTOR

The following adherent human and murine cell lines are seeded at 5 x $10^5$ cells/10 cm dish with 4 μg/ml polybrene: HT 1080 (ATCC No. CCL 121), Hela (ATCC No. CCL 2), BC10ME (Patek et al., Cell. Immuno. 72:113, 1982, ATCC No. TIB85), BCenv, BC10ME expressing HIV-1 IIIBenv (Warner et al., AIDS Res. and Human Retroviruses 7:645, 1991, L33 obtained from Gunther Dennert, University of Southern California, and L33env. The following day, 1.0 ml of filtered supernatant from the DA E3/19K pool is added to each of the cell culture plates. The following day, phleomycin is added to the media of all cell cultures. For cell lines that are already neomycin resistant the E3/19K in the KT-3C backbone (phleomycine resistant) is used. Transient supernatants for 293 2-3 or from DA derived lines can be used. The cultures are maintained until selection is complete and sufficient cell numbers are generated to test for gene expression. The transduced cell lines are designated HT 1080-E3/19K, Hela-E3/19K, BC10ME-E3/19K, L33-E3/19K and L33env-E3/19K respectively.

EBV transformed cell lines (BLCL), and other suspension cell lines, are transduced by co-cultivation with the irradiated producer cell line, DA-E3/19K. Specifically, irradiated (10,000 rads) producer line cells are plated at $5 \times 10^5$ cells /6 cm dish in growth media containing 4 μg/ml polybrene. After the cells have been allowed to attach for 2-24 hours, $10^6$ suspension cells are added. After 2-3 days, the suspension cells are removed, pelleted by centrifugation, resuspended in growth media containing 1mg/ml phleomycin, and seeded in 10 wells of a round bottom 96 well plate. The cultures were expanded to 24 well plates, then to T-25 flasks.

Example 11

EXPRESSION OF E3/19K IN THE MULTIVALENT RECOMBINANT RETROVIRAL VECTOR CONSTRUCT KT3B-GC/E3/19K

A. WESTERN BLOT ANALYSIS FOR E3/19K

Radio-immuno precipitation assay (RIPA) lysates are made from selected cultures for analysis of E3/19K expression. RIPA lysates are prepared from confluent plates of cells. Specifically, the media is first aspirated off the cells. Depending upon the size of the culture plate containing the cells, a volume of 100 to 500 μl ice cold RIPA lysis buffer (10 mM Tris, pH 7.4; 1% Nonidet P40 (Calbiochem, San Diego, CA); 0.1% SDS; 150 mM NaCl) is added to the cells. Cells are removed from plates using a micropipet and the mixture is transferred to a microfuge tube. The tube is centrifuged for 5 minutes to precipitate cellular debris and the supernatant is transferred to another tube. The supernatants are electrophoresed on a 10% SDS-PAGE gel and the protein bands are transferred to an Immobilon membrane in CAPS buffer (Aldrich, Milwaukee, WI) (10 mM CAPS, pH 11.0; 10% methanol) at 10 to 60 volts for 2 to 18 hours. The membrane is transferred from the CAPS buffer to 5% Blotto (5% nonfat dry milk; 50 mM Tris, pH 7.4; 150 mM NaCl; 0.02% sodium azide, and 0.05% Tween 20) and probed with a mouse monoclonal antibody to E3/19K (Severinsson et al., J. Cell Biol. 101:540, 1985). Antibody binding to the membrane is detected by the use of $^{125}$I-Protein A.

Example 12

FACS ANALYSIS OF E3/19K-VECTOR TRANSDUCED CELLS TO DEMONSTRATE DECREASED LEVELS OF CLASS I EXPRESSION COMPARED TO NON-TRANSDUCED CELLS.

Cell lines transduced with the E3/19K-vector are examined for MHC class I molecule expression by FACS analysis. Non-transduced cells are also analyzed for MHC class I molecule expression and compared with E3/19K transduced cells to determine the effect of transduction on MHC class I molecule expression.

Murine cell lines, L33-E3/19K, L33env-E3/19K, L33, L33env, BC10ME, BCenv, and BCenv-E3/19K, are tested for expression of the H-2D$^d$ molecule on the cell surface. Cells grown to subconfluent density are removed from culture dishes by treatment with Versene and washed two times with cold (4°C) PBS plus 1% BSA and 0.02% Na-azide (wash buffer) by centrifugation at 200g. Two x $10^6$ cells are placed in microfuge tubes and pelleted by centrifugation, 200g, and the supernatant is removed. Cell pellets are resuspended with the H-2D$^d$-specific Mab 34-2-12s (50μl of a 1:100 dilution of purified antibody, ATCC No. HB87) and incubated for 30 min at 4° C with occasional mixing. Antibody labeled cells are washed two times with 1 ml of wash buffer (4°C) centrifuged and the supernatant is removed. Cells are resuspended with a biotinylated goat anti-mouse kappa light chain Mab (Amersham, Arlington Heights, IL) (50μl, of a 1:100 dilution of purified antibody) and incubated for 30 min at 4°C. Cells are washed, resuspended with 50μl of avidin conjugated FITC (Pierce, Rockford, IL), and incubated for 30 min at 4°C. The cells are washed once more, resuspended in 1 ml of wash buffer, and held on ice prior to analysis on a FACStar Analyzer (Becton Dickinson, Los Angeles, CA). The mean fluorescence intensity of transduced cells is compared with that of non-transduced cells to determine the effect E3/19K protein has on surface MHC class I molecule expression.

Example 13

MURINE CTL ASSAY

Balb/c mice are injected with $10^7$ irradiated (10,000 rads) BCenv cells. After 7 days the spleens are harvested, dispersed into single cell suspension and $3 \times 10^6$ splenocytes/ml are cultured in vitro with $6 \times 10^4$ cells/ml irradiated BCenv or BCenv-E3/19K cells for 7 days at 37°C in T-25 flasks. Culture medium consists of RPMI 1640; 5% fetal bovine serum, heat-inactivated (FBS); 1 mM pyruvate; 50 μg/ml gentamicin and $10^{-5}$M 2-mercaptoethanol. Effector cells are harvested 7 days later and tested using various effector:target cell ratios in 96 well microtiter plates in a standard 4-6 hour assay. The assay employs $Na_2{}^{51}CrO_4$-labeled, 100 µCi, 1 hour at 37°C, (Amersham, Arlington Heights, Illinois) target cells (BC, BCenv, Warner et al., AIDS Res. and Human Retroviruses 7:645, 1991, or BCenv E3/19K) at 1.0 x $10^4$ cells/well with the final total volume per well of 200 µl. Following incubation, 100 µl of culture medium is removed and analyzed in a WALLAC gamma spectrometer (Gaithersburg, MD.). Spontaneous release (SR) is determined as counts per minute (CPM) CPM from targets plus medium and maximum release (MR) is determined asfrom targets plus 1M HCl. Percent target cell lysis is calculated as: [effector cell + target CPM) - (SR)]/[(MR) - (SR)] x 100 . Spontaneous release values of targets are typically 10%-30% of the MR. Tumor cells that have been transduced with the gene of interest (ribozyme, E3/19K, antisense, etc.) are used as stimulator and/or target cells in this assay to demonstrate the reduction of HIV-specific CTL induction and detection as compared to the non-transduced line which is the positive control.

Example 14

TUMOR REJECTION OF L33ENV CELLS BY BALB/C MICE IS ABROGATED WHEN CLASS I MOLECULE SURFACE EXPRESSION IS DECREASED BY THE E3-VECTOR TRANSDUCTION.

The L33env cell is being employed as a model for gene therapy treated transformed cells. Gene therapy treated cells produce a foreign protein making them possible targets for clearance by CTL. It has been demonstrated that Balb/c mice injected with live L33 tumor cells will develop a solid tumor identifiable by calliper measurement within three weeks post-exposure. However, Balb/c mice injected with live L33env transformed tumor cells (L33 cells transduced and selected for expression of the HIV-1$_{IIIB}$ envelope protein) recognize HIV env in the context of H-2D$^d$ and reject the tumor cells with no apparent tumor up to 15 weeks later (Warner et al., AIDS Res. and Human Retroviruses 7:645, 1991). Transformation of L33env cells with the E3/19K vector decreases cell surface expression of MHC class I molecules allowing these cells to evade immune surveillance and thereby establish a tumor. Development of an L33env tumor indicates that cell surface expression of MHC class I molecules has been decreased by cotransducing cells with the E19 gene. This impedes optimal immune system clearance mechanisms.

Three tumor cell lines L33, L33env, and L33env E3/19K are grown in DMEM containing 10% FBS. The tumor cells are gently rinsed with cold (4°C) PBS and treated with versene to remove them from the plate. After aspirating cells from plates, single cell suspensions are added to sterile plastic tubes. Cell suspensions are washed two times in sterile PBS (4°C), counted and resuspended in PBS to $10^7$ cells/ml. Balb/c mice (4-6 weeks old) are injected subcutaneous with $10^6$ live tumor cells (0.1 ml) and assessed for tumor formation and tumor clearance. Different mice are injected with different tumor cell lines. Mice injected with L33 cells are positive control animals for tumor formation while those injected with L33env are negative controls and should reject the tumor cells because of the env specific CTL response. The group of mice injected with E3/19K-transformed, L33env cells are monitored to show the effect that E3/19K expression in L33env cells has on the murine immune response to these tumor cells.

Example 15

FACS ANALYSIS OF E3/19K-VECTOR TRANSDUCED HUMAN CELLS TO DEMONSTRATE DECREASED LEVELS OF MHC CLASS I EXPRESSION COMPARED TO NON-TRANSDUCED CELLS.

Cell lines transduced with the E3/19K vector are examined for class I molecule expression by FACS analysis. Non-transduced cells are analyzed for class I molecule expression to compare with E3/19K transduced cells and determine the effect that transduction has on class I molecule expression.

Two human cell lines, JY-E3/19K and JY are tested for expression of the HLA-A2 molecule on the cell surface. Suspension cells grown to $10^6$ cells/ml are removed from culture flasks by pipet and washed two times with cold (4°C) PBS plus 1% BSA and 0.02% Na-azide (wash buffer) by centrifugation at 200g. Two million (2 x $10^6$) cells are placed in microfuge tubes, pelleted in at 200g, and the supernatant is removed. Cell pellets are resuspended with the HLA-A2-specific Mab BB7.2 (50µl of a 1:100 dilution of purified antibody, ATCC No. HB 82) and incubated with antibody for 30 min at 4°C with occasional mixing. Antibody labeled cells are washed two times with 1 ml of wash buffer (4°C). Prior to removing the supernatant, the cells are resuspended with a biotinylated rat anti-mouse kappa light chain Mab (50µl, of a 1:100 dilution of purified antibody) and incubated for 30 min at 4°C. Cells are washed, resuspended with 50µl of avidin conjugated FITC, and incubated for 30 min at 4°C. The cells are washed once more, and resuspended in 1 ml of wash buffer, and held on ice prior to analysis on a FACStar Analyzer. The mean fluorescence intensity of transduced cells is compared with that of non-transduced cells to determine the effect E3/19K protein has on surface MHC class I molecule expression.

Example 16

MEASUREMENT OF THE IMMUNE RESPONSE TO E3/19K-TRANSDUCED AND NONTRANSDUCED EBV-TRANSFORMED HUMAN JY CELLS BY HLA-A2 RESTRICTED, EBV-SPECIFIC HUMAN CTL LINES.

Human CTL lines propagated from donor blood samples using autologous EBV transformed cells as stimulators have been shown to be HLA-A2 restricted and specific for EBV proteins. These CTL lines, are propagated with autologous EBV transformed cells and can lyse JY target cells (HLA-A2$^+$ and EBV transformed). A chromium release assay can be performed with these CTL lines and JY target cells that have been transformed with the E3/19K gene or nontransduced. The E3/19K transformed JY target cell are used to demonstrate decreased recognition and lysis of this cell when compared to nontransformed JY target cells. These results indicate that cell transformation with agents that decrease MHC class I surface expression also decreases MHC class I restricted cell mediated immune responses in an *in vitro* human cell model system.

Approximately, 1 x 10$^6$ irradiated (10,000 rad) JY cells are cultured with 1 x 10$^7$ PBMC from a person that is HLA-A2 and verified to have an EBV response, in 10 mls of culture medium at 37°C 5% $CO_2$ for 7-10 days. The culture medium consists of RPMI 1640 supplemented with 5% heat inactivated fetal bovine serum preselected for CTL growth, 1 mM sodium pyruvate and nonessential amino acids. After the 7-10 day incubation the effector cells are harvested and tested in a standard 4-6 hour chromium release assay using $^{51}$Cr labelled JY cells as the positive control and $^{51}$Cr labelled JY-E3/19K. JY and JY-E3/19K cells are labelled with 300 μCi of $Na_2{}^{51}CrO_4$ for 1 hour at 37°C, then washed, counted, and used in the assay at 4 x 10$^3$ cells/well with the final total volume per well of 200 ul. Following incubation, 100 μl of culture medium is removed and analyzed in a WALLAC gamma spectrometer. Spontaneous release (SR) is determined as counts per minute (CPM) CPM from targets plus medium and maximum release (MR) is determined as from targets plus 1M HCl. Percent target cell lysis is calculated as: [effector cell + target CPM) - (SR)]/[(MR) - (SR)] x 100 . Spontaneous release values of targets are typically 10%-30% of the MR. BLCL cells that have been transduced with the gene of interest (ribozyme, E3/19K, antisense, etc.) are used as stimulator and/or target cells in this assay to demonstrate the reduction of EBV-specific CTL induction and detection as compared to the non-transduced line which is the positive control.

Example 17

*EX-VIVO* ADMINISTRATION OF A MULTIVALENT GLUCOCEREBROSIDASE RETROVIRAL VECTOR.

Pluripotent hematopoetic stem cells, CD34$^{+'}$ cells are collected from the bone marrow of a patient by a syringe evacuation performed by known techniques. Alternatively, CD34$^+$ cells may also be obtained from the cord blood of an infant if the patient is diagnosed before birth. Generally, 20 bone-marrow aspirations are obtained by puncturing femoral shafts or from the posterior iliac crest under local or general anesthesia. Bone marrow aspirations are then pooled and suspended in Hepes-buffered Hanks' balanced salt solution containing heperin sulfate at 100 U/ml and deoxyribonuclease I at 100 μg/ml and then subjected to a Ficoll gradient separation. The buffy coated marrow cells are then collected and washed according to CEPRATE™ LC (CD34) Separation system (Cellpro, Bothell, WA). The washed buffy coated cells are then stained sequentially with anti-CD34 monoclonal antibody, washed, then stained with biotinylated secondary antibody supplied with the CEPRATE™ system. The cell mixture is then loaded onto the CEPRATE™ avidin column. The biotin-labeled cells are adsorbed onto the column while unlabeled cells pass through. The column is then rinsed according to the CEPRATE™ system directions and CD34$^+$ cells eluted by agitation of the column by manually squeezing the gel bed. Once the CD34$^+$ cells are purified, the purified stem cells are counted and plated at a concentration of 1 x 10$^5$ cells/ml in Iscove's modified Dulbecco's medium, IMDM (Irvine Scientific, Santa Ana, CA) containing 20% pooled non-heat inactivated human AB serum (hAB serum).

After purification of CD34$^+$ cells, several methods of transforming purified stem cells may be performed. One approach involves transduction of the purified stem cell population with vector containing supernatant cultures derived from vector producing cells, Example 10. A second approach involves co-cultivation of an irradiated monolayer of vector producing cells with the purified population of non-adherent CD34$^+$ cells. A third and preferred approach involves a similar co-cultivation approach, however the purified CD34$^+$ cells are pre-stimulated with various cytokines and cultured 48 hours prior to the co-cultivation with the irradiated vector producing cells. Pre-stimulation prior to transduction increases effective gene transfer (Nolta et al., Exp. Hematol. 20:1065, 1992). The increased level of transduction is attributed to increased proliferation of the stem cells necessary for efficient retroviral transduction. Stimulation of these cultures to proliferate also provides increased cell populations for re-infusion into the patient.

Pre-stimulation of the CD34$^+$ cells is performed by incubating the cells with a combination of cytokines and growth factors which include IL-1, IL-3, IL-6 and mast cell growth factor (MGF). Pre-stimulation is performed by culturing 1-2 x 10$^5$ CD34$^+$ cells / ml of medium in T25 tissue culture flasks containing bone marrow stimulation medium for 48 hours. The bone marrow stimulation medium consists of IMDM containing 30% non-heat inactivated hAB serum, 2mM L-glutamine, 0.1 mM 2-mercaptoethanol, 1 μM hydrocortisone, and 1% deionized bovine serum albumin. All reagents used in the bone marrow cultures should be screened for their ability to support maximal numbers of granulocyte erythrocyte macrophage megakaryocyte colony-forming units from normal marrow. Purified recombinant human cytokines and growth factors (Immunex Corp., Seattle, WA) for pre-stimulation should be used at the following concentrations: *E. coli*-derived IL-1α (100 U/ml), yeast-derived IL-3 (5 ng/ml), IL-6 (50 U/ml), and MGF (50 ng/ml) (Anderson et al., Cell Growth Differ. 2:373, 1991).

After prestimulation of the CD34$^+$ cells, the cells are then transduced by co-cultivating on to the irradiated DA-based producer cell line, expressing the GC therapeutic multivalent vector, in the continued presence of the stimulation medium. The DA vector producing cell line is first trypsinized, irradiated using 10,000 rad and replated at 1-2 x 10$^5$/ml of bone marrow stimulation medium. The following day, 1-2 x10$^5$ prestimulated CD34$^+$ cells /ml were added onto the DA vector producing cell line monolayer followed by polybrene (Sigma, St. Louis, MO) to a final concentration of 4ug/ml. Co-cultivation of the cells should be performed for 48 hours. After co-cultivation , the CD34$^+$ cells are collected from the adherent DA vector producing cell monolayer by vigorous flushing with medium and plated for 2 hours to allow adherence of any dislodged vector producing cells. The cells are then collected and expanded for an additional 72 hours. The cells are collected and frozen in liquid nitrogen using a cryo-protectant in aliquots of 1 x 10$^7$ cells per vial. Once the transformed CD34$^+$ cells have been tested for the presence of adventitious agents, frozen transformed CD34$^+$ cells may be thawed, plated to a concentration of 1 x 10$^5$ cells/ml and cultured for an additional 48 hours in bone marrow stimulation medium. Transformed cells are then collected, washed twice and resuspended in normal saline. The number of transformed cells used to infuse back into the patient per infusion is projected to be at a minimum of 10$^7$ to 10$^8$ cells per patient per injection. The site of infusion may be directly into the patients bone marrow or i.v., into the peripheral blood stream. Patients receiving autologous transduced bone marrow cells may be either partially or whole body irradiated, to deplete existing bone marrow populations. Assessment of treatment may be performed at various time points, post infusion, by monitoring glucocerebrosidase activity in differentiated cell types and for length of expression. At the point when expression decreases or is non-existent, administration of transformed autologous cells may be re-injected into the patient

From the foregoing, it will be appreciated that, although specific embodiments of the invention have been described herein for purposes of illustration, various modifications may be made without deviating from the scope of the invention. Accordingly, the invention is not limited except as by the appended claims.

## Claims

1. A multivalent recombinant vector construct which directs the expression of a therapeutic protein and a second protein or active portion of the second protein capable of inhibiting MHC antigen presentation.

2. A multivalent recombinant vector construct as claimed in claim 1 which directs the expression of a therapeutic protein and a second protein or active portion of the second protein selected from the group consisting of E3/19K and H301.

3. A multivalent recombinant vector construct which directs the expression of a therapeutic protein and an antisense message that binds to a conserved region of MHC class I heavy chain transcripts or the transcript of a protein selected from β$_2$-microglobulin and PSF1.

4. A multivalent recombinant vector construct which directs the expression of a therapeutic protein and a ribozyme that cleaves a conserved region of MHC class I heavy chain transcripts or the transcript of a protein selected from β$_2$-microglobulin and PSF1.

5. A multivalent recombinant vector construct of any one of claims 1 to 4 which directs the expression of a therapeutic protein selected from the group consisting of factor IX, hemoglobulin, phenylalanine hydroxylase, adenosine deaminase, hypoxanthine-guanine phosphoribosyltransferase, dystrophin, the cystic fibrosis transmembrane conductance regulator and the liver receptor to LDL.

6. A multivalent recombinant vector construct of any one of claims 1 to 4 which directs the expression of factor VIII.

7. A multivalent recombinant vector construct of any one of claims 1 to 4 which directs the expression of α$_1$-antitrypsin.

8. A multivalent recombinant vector construct of any one of claims 1 to 4 which directs the expression of glucocere-

# EP 0 716 710 B1

brosidase.

9. A multivalent recombinant vector construct of any one of claims 1 to 8 which is a viral vector construct.

10. A multivalent recombinant vector construct as claimed in claim 9 which is a retroviral vector construct.

11. A virus carrying a vector construct according to claim 9 or claim 10.

12. A virus as claimed in claim 11 selected from the group consisting of poliovirus, rhinovirus, vaccinia virus, influenza virus, adenovirus, adeno-associated virus, herpes simplex virus and measles virus.

13. A virus as claimed in claim 11 selected from the group consisting of togaviridae, picornaviridae, poxviridae, adenoviridae, parvoviridae, herpesviridae, coronaviridae and paramyxoviridae viruses.

14. A virus as claimed in claim 11 which is a recombinant coronavirus.

15. A virus as claimed in claim 11 which is a recombinant Sindbis virus

16. A virus as claimed in claim 11 which is a recombinant retrovirus.

17. A pharmaceutical composition comprising a vector construct as claimed in any one of claims 1 to 10 or a virus as claimed in any one of claims 11 to 16 and a physiologically acceptable carrier or diluent.

18. *Ex vivo* tissue cells of an animal transformed with a multivalent recombinant vector construct according to any one of claims 1 to 10 whereby said therapeutic protein is expressed and an immune response against said cells *in vivo* expressing said therapeutic protein is suppressed.

19. Cells as claimed in claim 18 selected from the group consisting of fibroblast cells, bone marrow cells, endothelial cells, epithelial cells, muscle cells, neural cells, hepatocytes, thyroid follicular cells, hematopoietic progenitor cells and lymphocytes.

## Patentansprüche

1. Multivalentes rekombinantes Vektorkonstrukt, welches die Expression eines therapeutischen Proteins und eines zweiten Proteins oder eines aktiven Teils des zweiten Proteins, welches fähig ist, die MHC-Antigen-Präsentation zu inhibieren, steuert.

2. Multivalentes rekombinantes Vektorkonstrukt nach Anspruch 1, welches die Expression eines therapeutischen Proteins und eines zweiten Proteins oder eines aktiven Teils des zweiten Proteins, ausgewählt aus der Gruppe bestehend aus E3/19K und H301, steuert.

3. Multivalentes rekombinantes Vektorkonstrukt, welches die Expression eines therapeutischen Proteins und einer anti-Sinn-Message, die an eine konservierte Region der MHC-Klasse I schwere Ketten-Transkripte oder das Transkript eines Proteins, ausgewählt aus $\beta_2$-Mikroglobulin und PSF1, bindet, steuert.

4. Multivalentes rekombinantes Vektorkonstrukt, welches die Expression eines therapeutischen Proteins und eines Ribozyms, das eine konservierte Region von MHC-Klasse I-schwere Ketten-Transkripten oder des Transkripts eines Proteins, ausgewählt aus $\beta_2$-Mikroglobulin und PSF1, spaltet, steuert.

5. Multivalentes rekombinantes Vektorkonstrukt nach einem der Ansprüche 1 bis 4, welches die Expression eines therapeutischen Proteins, ausgewählt aus der Gruppe, bestehend aus Faktor IX, Hämoglobulin, Phenylalaninhydroxylase, Adenosindeaminase, Hypoxyanthin-Guanin-Phosphoribosyltransferase, Dystrophin, dem cystische Fibrosetransmembran-Leitfähigkeitsregulator und dem LDL-Rezeptor aus Leber, steuert.

6. Multivalentes rekombinantes Vektorkonstrukt nach einem der Ansprüche 1 bis 4, welches die Expression von Faktor VIII steuert.

7. Multivalentes rekombinantes Vektorkonstrukt nach einem der Ansprüche 1 bis 4, welches die Expression des $\alpha_1$-

24

Antitrypsins steuert.

8. Multivalentes rekombinantes Vektorkonstrukt nach einem der Ansprüche 1 bis 4, welches die Expression von Glucocerebrosidase steuert.

9. Multivaltentes rekombinantes Vektorkonstrukt nach einem der Ansprüche 1 bis 8, welches ein virales Vektorkonstrukt ist.

10. Multivalentes rekombinantes Vektorkonstrukt nach Anspruch 9, welches ein retrovirales Vektorkonstrukt ist.

11. Virus, der ein Vektorkonstrukt nach Anspruch 9 oder Anspruch 10 trägt.

12. Virus nach Anspruch 11, ausgewählt aus der Gruppe bestehend aus Poliovirus, Rhinovirus, Vacciniavirus, Influenzavirus, Adenovirus, Adeno-assoziiertem Virus, Herpes simplex-Virus und Masernvirus.

13. Virus nach Anspruch 11, ausgewählt aus der Gruppe bestehend aus Togaviridae- , Picornaviridae- , Poxyviridae- , Adenoviridae- , Parvoviridae- , Herpesviridae- , Coronaviridae- und Paramyxoviridae-Viren.

14. Virus nach Anspruch 11, welcher ein rekombinanter Coronavirus ist.

15. Virus nach Anspruch 11, welcher ein rekombinanter Sind- bis-Virus ist.

16. Virus nach Anspruch 11, welcher ein rekombinanter Retrovirus ist.

17. Pharmazeutische Zusammensetzung, umfassend ein Vektorkonstrukt nach einem der Ansprüche 1 bis 10 oder ein Virus nach einem der Ansprüche 11 bis 16 und einen physiologisch annehmbaren Träger oder ein physiologisch annehmbares Verdünnungsmittel.

18. *Ex vivo*-Gewebezellen eines Tieres, die mit einem multivalenten rekombinanten Vektorkonstrukt nach einem der Ansprüche 1 bis 10 transformiert sind, wobei das therapeutische Protein exprimiert wird und eine Immunantwort gegen diese Zellen *in vivo* bei Expression dieses therapeutischen Proteins unterdrückt ist.

19. Zellen nach Anspruch 18, ausgewählt aus der Gruppe bestehend aus Fibroblastenzellen, Knochenmarkszellen, Endothelzellen, Epithelzellen, Muskelzellen, Nervenzellen, Leberzellen, follekularen Schilddrüsenzellen, hematopoetischen Vorläuferzellen und Lymphocyten.

**Revendications**

1. Construction de vecteur recombinant polyvalent qui commande l'expression d'une protéine thérapeutique et d'une seconde protéine ou d'une portion active de la seconde protéine capable d'inhiber la présentation d'un antigène par le CMH.

2. Construction de vecteur recombinant polyvalent telle que revendiquée à la revendication 1 qui commande l'expression d'une protéine thérapeutique et d'une seconde protéine ou d'une portion active de la seconde protéine choisie dans le groupe constitué de E3/19K et H301.

3. Construction de vecteur recombinant polyvalent qui commande l'expression d'une protéine thérapeutique et d'un message antisens qui se lie à une région conservée de transcrits de chaîne lourde du CMH de classe I ou au transcrit d'une protéine choisie parmi la microglobuline $\beta_2$ et PSF1.

4. Construction de vecteur recombinant polyvalent qui commande l'expression d'une protéine thérapeutique et d'un ribozyme qui clive une région conservée de transcrits de chaîne lourde du CMH de classe I ou le transcrit d'une protéine choisie parmi la microglobuline $\beta_2$ et PSF1.

5. Construction de vecteur recombinant polyvalent de l'une quelconque des revendications 1 à 4 qui commande l'expression d'une protéine thérapeutique choisie dans le groupe constitué du facteur IX, de l'hémoglobine, de la phénylalanine hydroxylase, de l'adénosine désaminase, de l'hypoxanthine-guanine phosphoribosyltransférase, de la dystrophine, du CFTR (Cystic Fibrosis Transmembrane conductance Regulator) et du récepteur hépatique des LDL.

6. Construction de vecteur recombinant polyvalent selon l'une quelconque des revendications 1 à 4 qui commande l'expression du facteur VIII.

7. Construction de vecteur recombinant polyvalent selon l'une quelconque des revendications 1 à 4 qui commande l'expression de l'$\alpha_1$-antitrypsine.

8. Construction de vecteur recombinant polyvalent selon l'une quelconque des revendications 1 à 4 qui commande l'expression de la glucocérébrosidase.

9. Construction de vecteur recombinant polyvalent selon l'une quelconque des revendications 1 à 8 qui est une construction de vecteur viral.

10. Construction de vecteur recombinant polyvalent telle que revendiquée à la revendication 9 qui est une construction de vecteur rétroviral.

11. Virus portant une construction de vecteur selon la revendication 9 ou la revendication 10.

12. Virus tel que revendiqué à la revendication 11 choisi dans le groupe constitué des virus poliomyélitique, rhinovirus, virus de la vaccine, virus de la grippe, adénovirus, virus adéno-associé, virus de l'Herpes simples et virus de la rougeole.

13. Virus tel que revendiqué à la revendication 11 choisi dans le groupe constitué des virus togaviridés, picornaviridés, poxviridés, adénoviridés, parvoviridés, herpesviridés, coronaviridés, paramyxoviridés.

14. Virus tel que revendiqué à la revendication 11 qui est un coronavirus recombinant.

15. Virus tel que revendiqué à la revendication 11 qui est un virus sindbis recombinant.

16. Virus tel que revendiqué à la revendication 11 qui est un rétrovirus recombinant.

17. Composition pharmaceutique comprenant une construction de vecteur telle que revendiquée dans l'une quelconque des revendications 1 à 10 ou un virus tel que revendiqué dans l'une quelconque des revendications 11 à 16 et un véhicule ou diluant physiologiquement acceptable.

18. Culture de tissu *ex vivo* d'un animal transformé avec une construction de vecteur recombinant polyvalent selon l'une quelconque des revendications 1 à 10 dans laquelle ladite protéine thérapeutique est exprimée et une réponse immunitaire contre lesdites cellules exprimant *in vivo* ladite protéine thérapeutique est supprimée.

19. Cellules telles que revendiquées à la revendication 18 choisies dans le groupe constitué de fibroblastes, de cellules de moelle osseuse, de cellules endothéliales, de cellules épithéliales, de cellules musculaires, de cellules nerveuses, d'hépatocytes, de cellules folliculaires thyroïdiennes, de cellules souches hématopoïétiques et de lymphocytes.